(19) Europäisches Patentamt
European Patent Office
Office européen des brevets

(11) **EP 4 020 484 A1**

(12) **EUROPEAN PATENT APPLICATION**
published in accordance with Art. 153(4) EPC

(43) Date of publication:
**29.06.2022 Bulletin 2022/26**

(21) Application number: **20854906.3**

(22) Date of filing: **19.08.2020**

(51) International Patent Classification (IPC):
**G16B 20/20** (2019.01)      **G16B 15/00** (2019.01)
**G16B 30/10** (2019.01)      **G16B 40/20** (2019.01)

(52) Cooperative Patent Classification (CPC):
**G16B 15/00; G16B 20/20; G16B 30/10;
G16B 40/20**

(86) International application number:
**PCT/KR2020/010853**

(87) International publication number:
**WO 2021/034034 (25.02.2021 Gazette 2021/08)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **19.08.2019   KR 20190101246**
**05.12.2019   KR 20190160407**
**18.08.2020   KR 20200103240**

(71) Applicant: **Green Cross Genome Corporation
Yongin-si, Gyeonggi-do 16924 (KR)**

(72) Inventors:
 • **KI, Chang-Seok**
   **Yongin-si Gyeonggi-do 16924 (KR)**
 • **CHO, Eun Hae**
   **Yongin-si Gyeonggi-do 16924 (KR)**
 • **LEE, Junnam**
   **Yongin-si Gyeonggi-do 16924 (KR)**

(74) Representative: **Mewburn Ellis LLP
Aurora Building
Counterslip
Bristol BS1 6BX (GB)**

(54) **METHOD FOR DETECTING CHROMOSOMAL ABNORMALITY BY USING INFORMATION ABOUT DISTANCE BETWEEN NUCLEIC ACID FRAGMENTS**

(57)    The present invention relates to a method for detecting chromosomal abnormality by using information about the distance between nucleic acid fragments and, more particularly, to a method for detecting chromosomal abnormality by using a method, which extracts a nucleic acid from a biological sample so as to acquire sequence information, and then calculate the distance between nucleic acid fragment Representative Positions. A method for determining chromosomal abnormality, according to the present invention, uses a method, which analyzes and uses, unlike a method using a step of determining a chromosomal quantity on the basis of a conventional read count, the concept of the distance between aligned nucleic acid fragments and thus the conventional method has decreasing accuracy when the read count decreases. However, the method of the present invention is useful since the accuracy of detection can increase even if the read count decreases, and the accuracy of the detection is high even if the distance between nucleic acid fragments in a predetermined section instead of an entire chromosomal section is analyzed.

FIG. 1

FIG. 2

**Description**

[Technical Field]

The present invention relates to a method for detecting a chromosomal abnormality using information about distances between nucleic acid fragments, and more specifically to a method for detecting a chromosomal abnormality by extracting nucleic acids from a biological sample to obtain sequence information and then calculating the distance between Representative Positions of nucleic acid fragments.

[Background Art]

Chromosomal abnormalities are associated with genetic defects and tumor-related diseases. The term "chromosomal abnormality" may mean deletion or duplication of chromosomes, deletion or duplication of a portion of chromosomes, or a break, translocation, or inversion in chromosomes. A chromosomal abnormality is a disorder related to genetic balance and may cause fetal mortality or serious defects in physical and mental condition, as well as tumor-related diseases. For example, Down's syndrome is a common chromosome number abnormality caused by the presence of a third copy of chromosome 21 (which is also called "trisomy 21"). Edwards syndrome (trisomy 18), Patau syndrome (trisomy 13), Turner syndrome (XO), and Klinefelter syndrome (XXY) are also chromosomal abnormalities. Chromosomal abnormalities are also found in tumor patients. For example, duplication of chromosomes 4q, 11q, and 22q and deletion of chromosome 13q were observed in liver cancer patients (liver adenomas and adenocarcinomas patents), and duplication of chromosomes 2p, 2q, 6p, and 11q, and deletion of chromosomes 6q, 8p, 9p, and 21 were observed in pancreatic cancer patients. These chromosomal regions are associated with tumor-related oncogene and tumor suppressor gene regions.

Chromosomal abnormalities can be detected using karyotype and FISH (fluorescent in-situ hybridization). This detection method is disadvantageous in terms of time, effort, and accuracy. In addition, DNA microarrays can be used to detect chromosomal abnormalities. In particular, a genomic DNA microarray system is capable of easily producing a probe and detecting chromosomal abnormalities in the intron region of the chromosome as well as the extended region of the chromosome, but is difficult to use to produce a large number of DNA fragments, the chromosomal locations and functions of which have been identified.

Recently, next-generation sequencing has been used to analyze chromosome number abnormalities (Park, H., Kim et al., Nat Genet 2010, 42, 400-405.; Kidd, J.M. et al., Nature 2008, 453, 56-64). However, this technology requires high coverage readings for the analysis of chromosome number abnormalities (aneuploidy), and CNV measurements also require independent validation. Therefore, this technology was unsuitable for general gene search analysis in the prior art because it is expensive and the results thereof are difficult to understand.

Real-time qPCR is currently used as a state-of-the-art technique for quantitative genetic analysis, because a wide kinetic range (Weaver, S. et al, Methods 2010, 50, 271-276) and a linear relationship between threshold cycle and initial target amount (Deepak, S. et al., Curr Genomics 2007, 8, 234-251) are reproducibly observed. However, the qPCR assay is not sensitive enough to distinguish between different numbers of copies.

Meanwhile, conventional prenatal test items for fetal chromosomal abnormalities include ultrasound examination, blood marker tests, amniocentesis, chorionic examination, transdermal umbilical cord blood tests and the like (Mujezinovic F. et al., Obstet. Gynecol. 2007, 110(3):687-94.). Among them, ultrasound examination and blood marker tests are classified as screening tests, and amniocentesis is classified as a confirmatory test. Ultrasound examination and blood marker tests, which are non-invasive methods, are safe because they do not include directly collecting samples from the fetus, but the sensitivity of the tests is decreased to 80% or less (ACOG Committee on Practice Bulletins. 2007). Invasive methods such as amniocentesis, chorionic examination, and transdermal umbilical cord blood tests are capable of detecting fetal chromosomal abnormalities, but have a disadvantage in that there is the possibility of loss of the fetus due to the invasive medical procedure.

Lo et al. succeeded in sequencing the Y chromosome of a fetal genetic material from maternal plasma and serum in 1997 and then used the fetal genetic material derived from the mother for prenatal testing (Lo Y. M., et al. The Lancet. 1997, 350 (9076):485-7). The fetal genetic material that is present in the maternal blood is some trophoblast cells that undergo apoptosis during placental remodeling and enter the maternal blood through a substance exchange mechanism, is actually derived from the placenta and is defined as "cff DNA (cell-free fetal DNA)".

cff DNA is detected as early as day 18 of embryo transfer and is found in most maternal blood at day 37 of embryo transfer. cff DNA is a short strand of 300 bp or less and is present in a small amount in maternal blood, so massive parallel sequencing technology using next-generation sequencing (NGS) is used to apply the same to detect fetal chromosomal abnormalities. Non-invasive fetal chromosomal abnormality detection using massive parallel sequencing has detection sensitivity of 90-99% or more depending on the chromosome, but causes false-positive and false-negative results in 1 to 10% of cases, and thus requires correction therefor (Gil MM, et al. Ultrasound Obstet.

Gynecol. 2015, 45(3):249-66).

Accordingly, as a result of extensive and earnest efforts to solve the above problems and develop a method for detecting chromosomal abnormalities with high sensitivity and accuracy, the present inventors found that chromosomal abnormalities can be detected with high sensitivity and accuracy by grouping nucleic acid fragments aligned in chromosomal regions, calculating the distance between the nucleic acid fragment Representative Positions, and comparing the distance from the normal group, and the present invention has been completed based on these findings.

[Disclosure]

Therefore, it is one object of the present invention to provide a method for determining a chromosomal abnormality using information on the distance between nucleic acid fragments.

It is another object of the present invention to provide a device for determining a chromosomal abnormality using information on the distance between nucleic acid fragments.

It is another object of the present invention to provide a computer-readable storage medium including instructions configured to be executed by a processor for determining a chromosomal abnormality by the method described above.

In accordance with one aspect of the present invention, the above and other objects can be accomplished by the provision of a method of detecting a chromosomal abnormality, the method comprises: calculating a distance between Representative Positions of nucleic acid fragments extracted from a biological sample.

In accordance with another aspect of the present invention, provided is a device for determining a chromosomal abnormality, the device including a decoder configured to extract nucleic acids from a biological sample and decode sequence information, an aligner configured to align the decoded sequence to a reference genome database, and a chromosomal abnormality determiner configured to measure the distance between the Representative Positions of the aligned nucleic acid fragments among the selected nucleic acid fragments to thereby calculate a fragment distance (FD), to calculate a fragment distance index (FDI) over the entire chromosomal region or each specific genomic region based on the calculated FD, and to determine that there is a chromosomal abnormality when the FDI does not fall within a cutoff value range.

In accordance with another aspect of the present invention, provided is a computer-readable storage medium including an instruction configured to be executed by a processor for detecting a chromosomal abnormality through the following steps including:

(A) extracting nucleic acids from a biological sample and obtaining nucleic acid fragments to obtain sequence information;

(B) aligning the nucleic acid fragments to a reference genome database based on the obtained sequence information (reads);

(C) measuring the distance between the Representative Positions of the selected nucleic acid fragments to calculate a fragment distance (FD); and

(D) calculating a fragment distance index (FDI) over the entire chromosomal region or in each specific genomic region based on the FD calculated in step (C) and determining that there is a chromosomal abnormality when the FDI does not fall within a cutoff value range.

In accordance with another aspect of the present invention, provided is a method for detecting a chromosomal abnormality, the method including: (A) extracting nucleic acids from a biological sample to obtain sequence information (reads); (B) aligning the obtained reads to a reference genome database; (C) measuring the distance between the aligned reads in the aligned sequence information (reads) to calculate a read distance (RD); and (D) calculating a read distance index (RDI) over the entire chromosomal region or in each specific genomic region based on the RD calculated in step (C), and determining that there is a chromosomal abnormality when the RDI does not fall within a cutoff value range.

In accordance with another aspect of the present invention, provided is a device for determining a chromosomal abnormality, including a decoder configured to extract nucleic acids from a biological sample and decode sequence information, an aligner configured to align the decoded sequence to a reference genome database, and a chromosomal abnormality determiner configured to measure the distance between the aligned reads among the selected sequence information (reads) to calculate a read distance (RD), to calculate a read distance index (RDI) over the entire chromosomal region or each specific genomic region based on the calculated RD, and to determine that there is a chromosomal abnormality when the RDI does not fall within a cutoff value range.

In accordance with another aspect of the present invention, provided is a computer-readable storage medium including instructions configured to be executed by a processor for detecting a chromosomal abnormality through the following steps, including: (A) extracting nucleic acids from a biological sample to obtain sequence information; (B) aligning the obtained sequence information (reads) to a reference genome database; (C) measuring the distance between the aligned reads in the aligned sequence information (reads) to calculate a read distance (RD); and (D) calculating a read distance index (RDI) over the entire chromosomal region or in each specific genomic region based on the RD calculated in step (C) and determining that there is a chromosomal abnormality when the RDI does not fall within a cutoff value range.

[Description of Drawings]

FIG. 1 is an overall flowchart for determining a chromosomal abnormality based on FD according to one embodiment of the present invention.

FIG. 2 is a conceptual diagram illustrating a method of calculating the FD of the present invention from reads produced using a single-end sequencing method.

FIG. 3 is a conceptual diagram illustrating a method of calculating the FD of the present invention from reads produced using a paired-end sequencing method.

FIG. 4 is a conceptual diagram illustrating a method of correcting an FD using location information other than reads in the present invention.

FIG. 5 is a graph showing the difference between FD calculated using position information other than reads and FD calculated without using the same, based on read data produced by a paired-end sequencing method according to an embodiment of the present invention.

FIG. 6 is an overall flowchart for determining a chromosomal abnormality based on an RD according to an embodiment of the present invention.

FIG. 7 is a conceptual diagram illustrating the read distance calculated in the RD-based method according to an embodiment of the present invention, wherein the reads used to calculate the read distance may be used regardless of the aligned direction (FIG. 7A), and may be used in consideration of the aligned direction (positive direction: FIG. 7B, negative direction: FIG. 7C).

FIG. 8 is a diagram illustrating the read count of the X chromosome and the distribution of RepRD in the RD-based method according to an embodiment of the present invention, which indicates that the relationship between the two values is nonlinear rather than linear.

FIG. 9 is a diagram illustrating the read count of each chromosome and the distribution of RepRD in the RD-based method according to an embodiment of the present invention, wherein (A) is a normal chromosome, (B) is trisomy 21, (C) is trisomy 18, and (D) is trisomy 13.

FIG. 10 illustrates the relationship between an RDI calculated by the RD-based method according to an embodiment of the present invention, and each of fetal fraction (FIG. 10A), gestational weeks (FIG. 10B), and G-score (Korea Patent No. 10-1686146, FIG. 10C).

FIG. 11 illustrates the result of ROC analysis of a normal group and each sample identified to have a chromosome aneuploidy in the RD-based method according to an embodiment of the present invention.

FIG. 12 illustrates the result of confirming accuracy as a function of the number of reads in the RD-based method according to an embodiment of the present invention, wherein the X axis represents the number of reads and the Y axis represents the AUC.

FIG. 13 illustrates the result of confirming the relationship between the RD-based method according to an embodiment of the present invention and the number of reads and chromosomal abnormalities.

FIG. 14 illustrates the result of a comparison between the RD-based method according to an embodiment of the present invention and microarray analysis.

FIG. 15 illustrates the result of confirming the distribution of RDI in which the RepRD of a normal subject and a chromosome 21 aneuploidy sample is set as the reciprocal of the median in the RD-based method according to an embodiment of the present invention.

FIG. 16 illustrates the result of confirming the distribution of RDI in which RepRD of a normal subject and a chromosome 21 aneuploidy sample is set as a mean in the RD-based method according to an embodiment of the present invention.

FIG. 17 illustrates the result of confirming the distribution of RDI in which RepRD of a normal subject and a chromosome 21 aneuploidy sample is set as the reciprocal of the mean in the RD-based method according to an embodiment of the present invention.

[Best Mode]

Unless defined otherwise, all technical and scientific terms used herein have the same meanings as appreciated by those skilled in the field to which the present invention pertains. In general, the nomenclature used herein is well-known in the art and is ordinarily used.

It was found in the present invention that a chromosomal abnormality can be detected with high sensitivity and accuracy by aligning sequence information (read) data obtained from a sample to a reference genome, grouping the aligned nucleic acid fragments, calculating the distance between the nucleic acid fragment Representative Positions, and comparing a representative value of the chromosome to be analyzed for a normal group with that of a test subject.

The method for detecting a chromosomal abnormality according to the present invention can be used not only for fetal chromosomal abnormalities such as aneuploidy, but also for tumor detection, i.e., tumor diagnosis or prognosis.

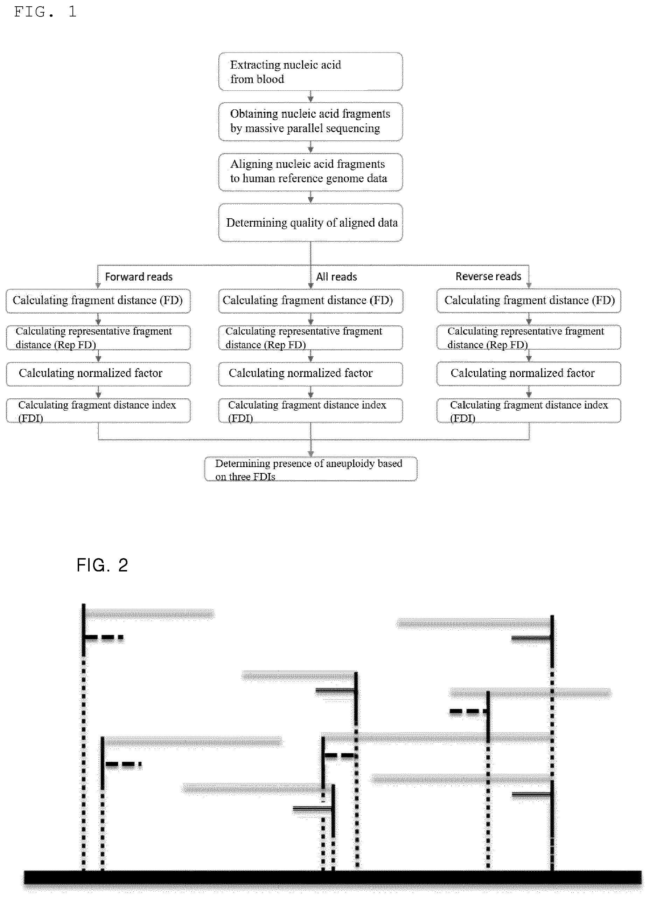

That is, in the method according to one embodiment of the present invention, DNA extracted from blood was sequenced, the result was aligned to a reference genome, nucleic acid fragments were grouped into whole, forward and reverse groups, the distance between nucleic acid fragment Representative Positions (fragment distance, FD) for each group was calculated, a representative FD (RepFD) of the distance between the Representative Positions of nucleic acid fragments was derived for each genomic region, an RepFD ratio was calculated using a normalized factor, the calculated RepFD ratio was compared with an RepFD ratio in a reference group having normal subjects to obtain a fragment distance index (FDI) for each group, and it was determined that the test subject had a chromosomal abnormality when the FDI for each group was less than or greater than the cutoff value (FIG. 1).

In one aspect, the present invention is directed to a method of detecting a chromosomal abnormality, the method comprises: calculating a distance between Representative Positions of nucleic acid fragments extracted from a biological sample.

In the present invention, any nucleic acid fragment can be used without limitation, as long as it is a fragment of a nucleic acid extracted from a biological sample, and is preferably a fragment of a cell-free nucleic acid or an intracellular nucleic acid, but is not limited thereto.

In the present invention, the nucleic acid fragment may be obtained by direct sequencing, next-generation sequencing, or sequencing through non-specific whole genome amplification.

In the present invention, the direct sequencing of the nucleic acid fragment may be performed using any known technique.

In the present invention, the sequencing through non-specific whole genome amplification includes any method including amplifying nucleic acids using random primers and then performing sequencing.

In the present invention, the method including calculating the distance between the Representative Positions of nucleic acid fragments using next-generation sequencing and determining whether or not there is a chromosomal abnormality based thereon is carried out by a method including the following steps:

(A) extracting nucleic acids from a biological sample and obtaining nucleic acid fragments to obtain sequence information (reads) therefrom;

(B) identifying the positions of the nucleic acid fragments in a reference genome database based on the obtained sequence information (reads);

(C) grouping the sequence information (reads) into whole sequences, forward sequences and reverse sequences;

(D) defining Representative Positions of the respective nucleic acid fragments using the grouped sequence information, and measuring the distance between the Representative Positions to calculate a fragment distance (FD) for each group; and

(E) calculating a fragment distance index (FDI) for the entire chromosomal region or each specific region based on the FD for each group calculated in step (D) and determining that there is a chromosomal abnormality when the FDI does not fall within a cutoff value range.

As used herein, the term "chromosomal abnormality" is defined to include a variety of variations occurring in chromosomes, and may be broadly divided into number abnormalities, structural abnormalities, microdeletions, chromosomal instability, and the like.

An chromosome number abnormality refers to a case in which an abnormality occurs in the number of chromosomes, and may, for example, include all cases where an abnormality occurs in 23 pairs and a total of 46 chromosomes, such as Down's syndrome (in which the total number of chromosomes is 47, including one extra copy of chromosome 21), Turner syndrome (in which the total number of chromosomes is 45 due to the presence of only one X chromosome) and Klinefelter syndrome (having an abnormal number of chromosomes such as XXYY, XXXY, and XXXXY).

Structural chromosomal abnormalities refer to all cases in which there is no change in the number of chromosomes but there is a change in the structure of chromosomes, such as deletions, duplications, inversions, translocations, fusions, and microsatellite instability (MSI-H). Examples thereof include partial deletion of chromosome 5 (cat's cry syndrome), partial deletion of chromosome 7 (Phillips syndrome), partial duplication of chromosome 12 (Wolf-Hirschhorn syndrome), and the like. Structural chromosomal abnormalities found in tumor patients include translocation between chromosomes 9 and 22 (chronic myelogenous leukemia), duplication of chromosomes 4q, 11q, and 22q and deletion of chromosome 13q (liver cancer), duplication of chromosomes 2p, 2q, 6p, and 11q, and deletion of chromosomes 6q, 8p, 9p, and 21 (pancreatic cancer), TMPRSS2-TRG gene fusion (prostate cancer), and microsatellite instability over the overall chromosomes (colon cancer). These regions are related to regions of oncogenes and tumor suppressor genes associated with tumors, but the invention is not limited thereto.

In the present invention,
the step (A) includes:

(A-i) obtaining nucleic acids from blood, semen, vaginal cells, hair, saliva, urine, oral cells, amniotic fluid containing placental cells or fetal cells, tissue cells, and a mixture thereof;

(A-ii) removing proteins, fats, and other residues from the collected nucleic acids using a salting-out method, a column chromatography method, or a bead method to obtain purified nucleic acids;

(A-iii) producing a single-end sequencing or pairend sequencing library for the purified nucleic acids or nucleic acids randomly fragmented by an enzymatic digestion, pulverization, or hydroshear method;

(A-iv) reacting the produced library with a next-generation sequencer; and

(A-v) obtaining sequence information (reads) of the nucleic acids in the next-generation sequencer.

In the present invention, the next-generation sequencer may be used for any sequencing method known in the art. Sequencing of nucleic acids isolated using the selection method is typically performed using next-generation sequencing (NGS). Next-generation sequencing includes any sequencing method that determines the nucleotide sequence either of each nucleic acid molecule or of a proxy clonally expanded from each nucleic acid molecule in a highly similar manner (e.g., $10^5$ or more molecules are sequenced simultaneously). In one embodiment, the relative abundance of nucleic acid species in the library can be estimated by counting the relative number of occurrences of the sequence homologous thereto in data produced by sequencing experimentation. Next-generation sequencing is known in the art and is described, for example, in Metzker, M. (2010), Nature Biotechnology Reviews 11:31-46, which is incorporated herein by reference.

In one embodiment, next-generation sequencing is performed to determine the nucleotide sequence of each nucleic acid molecule (using, for example, a HelioScope Gene-Sequencing system from Helicos Biosciences or a PacBio RS system from Pacific Biosciences). In other embodiments, massive parallel short-read sequencing, which produces more bases of the sequence per sequencing unit than other sequencing methods, for example, other sequencing methods that produce fewer but longer reads, determines the nucleotide sequence of a proxy clonally expanded from each nucleic acid molecule (using, for example, a Solexa sequencer from Illumina Inc., located in San Diego, CA; 454 Life Sciences (Branford, Connecticut) and Ion Torrent). Other methods or devices for next-generation sequencing may be provided by 454 Life Sciences (Branford, Connecticut), Applied Biosystems (Foster City, CA; SOLiD Sequencer), Helicos Biosciences Corporation (Cambridge, MA) and emulsion and microfluidic sequencing nanodrops (e.g., GnuBIO Drops), but are not limited thereto.

Platforms for next-generation sequencing include, but are not limited to, the Roche/454's genome sequencer (GS) FLX System, Illumina/Solexa genome analyzer (GA), Life/APG's Support Oligonucleotide Ligation Detection (SOLiD) system, Polonator's G.007 system, Helicos Biosciences' HelioScope Gene-Sequencing system, Pacific Biosciences' PacBio RS system, and MGI's DNBseq.

NGS technologies may, for example, include one or more of template production, sequencing, imaging, and data analysis steps.

Template production step. Methods for producing templates include randomly disrupting nucleic acids (e.g., genomic DNA or cDNA) into small sizes and producing sequencing templates (e.g., fragment templates or mate-pair templates). Spatially separated templates may be attached or immobilized on a solid surface or support, which allows simultaneous large-scale sequencing reactions to be performed. Examples of types of templates that can be used for NGS reactions include templates amplified from clones derived from single DNA molecules and single DNA molecule templates.

Methods for producing the templates amplified from clones include, for example, emulsion PCR (emPCR) and solid-phase amplification.

EmPCR may be used to produce templates for NGS. Typically, a library of nucleic acid fragments is produced, and adapters containing universal priming sites are ligated to the ends of the fragments. The fragments are then denatured into single strands and captured by beads. Each bead captures a single nucleic acid molecule. After amplification and enrichment of emPCR beads, a large amount of templates can be attached, immobilized to a polyacrylamide gel on a standard microscope slide (from, for example, Polonator) and chemically crosslinked to an amino-coated glass surface (e.g., Life/APG; Polonator), or deposited in individual PicoTiterPlate (PTP) wells (e.g., Roche/454). At this time, an NGS reaction may be performed.

Solid-phase amplification can also be used to produce templates for NGS. Typically, the front and rear primers are covalently attached to the solid support. The surface density of the amplified fragment is defined as the ratio of primer to template on the support. Solid-phase amplification is capable of producing millions of spatially separated template clusters (e.g., Illumina/Solexa). The ends of the template cluster can be hybridized to universal primers for NGS reactions.

Other methods for producing clone-amplified templates include, for example, multiple displacement amplification (MDA) (Lasken R. S.; Curr Opin Microbiol. 2007; 10(5):510-6) . MDA is a non-PCR-based DNA amplification method. The reaction involves annealing random hexamer primers to templates and synthesizing DNA by a high-fidelity enzyme, typically Φ29 polymerase, at a constant temperature. MDA can yield large-scale products with a lower error frequency.

Template amplification methods such as PCR can bind the NGS platform to the target or enrich specific regions of the genome (e.g., exons). Representative template enrichment methods include, for example, microdroplet PCR (Tewhey R. et al., Nature Biotech. 2009, 27:1025-1031), custom-designed oligonucleotide microarrays (e.g., Roche/NimbleGen oligonucleotide microarrays), solutionbased hybridization (e.g., molecular inversion probes, MIPs) (Porreca GJ et al., Nature Methods, 2007, 4:931-936; Krishnakumar S. et al., Proc. Natl. Acad. Sci. USA, 2008, 105:9296-9310; Turner EH et al., Nature Methods, 2009, 6:315-316), and biotinylated RNA capture sequences (Gnirke A. et al., Nat. Biotechnol. 2009;27(2):182-9).

Single-molecule templates are another type of template that can be used for NGS reactions. Spatially separated single-molecule templates may be immobilized on a solid support by a variety of methods. In one approach, each primer molecule is covalently attached to a solid support. The adapter is added to the template and the template is then hybridized to the immobilized primer. In another approach, a single-molecule template is covalently attached to a solid support by priming and extending a single-stranded single-molecule template from the immobilized primer. The universal primer is then hybridized to the template. In another approach, a single polymerase molecule is attached to a solid support to which a primed template is attached.

Sequencing and imaging. Representative sequencing and imaging methods for NGS include, but are not limited to, cyclic reversible termination (CRT), sequencing by ligation (SBL), pyrosequencing and real-time sequencing.

CRT uses reversible terminators in a cyclic method minimally including nucleotide incorporation, fluorescence imaging and cleavage steps. Typically, DNA polymerases incorporates a single fluorescently modified nucleotide complementary to the complementary nucleotide of the template base in the primer. DNA synthesis is terminated after incorporation of a single nucleotide, and the unincorporated nucleotides are washed out. Imaging is performed to determine the homology of the incorporated labeled nucleotides. Then, in the cleavage step, the terminator/inhibitor and the fluorescent dye are removed. Representative NGS platforms using the CRT method include, but are not limited to, Illumina/Solexa Genome Analyzer (GA) using a clone-amplification template method combined with a 4-color CRT method detected by total internal reflection fluorescence (TIRF); and Helicos Biosciences/HelioScope using a single-molecule template method combined with a 1-color CRT method detected by TIRF.

SBL uses a DNA ligase and either a 1-base-encoded probe or a 2-base-encoded probe for sequencing.

Typically, a fluorescently labeled probe is hybridized to a complementary sequence adjacent to the primed template. DNA ligases are used to ligate dye-labeled probes to primers. After the non-ligated probes are washed, fluorescence imaging is performed to determine the identity of the ligated probes. The fluorescent dye may be removed using a cleavable probe that regenerates the 5'-PO4 group for subsequent ligation cycles. Alternatively, new primers may be hybridized to the template after old primers have been removed. Representative SBL platforms include, but are not limited to, Life/APG/SOLiD (support oligonucleotide ligation detection), which uses a two-base-encoded probe.

The pyrosequencing method is based on detection of the activity of DNA polymerase with another chemiluminescent enzyme. Typically, this method includes sequencing a single strand of DNA by synthesizing the complementary strand along one base pair at a time and detecting the base that is actually added at each step. The template DNA is stationary, and solutions of A, C, G, and T nucleotides are sequentially added and removed during the reaction. Light is generated only when the nucleotide solution replenishes the unpaired base of the template. The sequence of the solution generating the chemiluminescent signal is used to determine the sequence of the template. Representative pyrosequencing platforms include, but are not limited to, Roche/454 using DNA templates produced from 1 to 2 million beads deposited in PTP wells by emPCR.

Real-time sequencing involves imaging the continuous incorporation of dye-labeled nucleotides during DNA synthesis. Representative real-time sequencing platforms include, but are not limited to, Pacific Biosciences' platform using DNA polymerase molecules attached to the surface of respective zero-mode waveguide (ZMW) detectors for obtaining sequence information when phosphate-linked nucleotides are incorporated in the growing primer strands; the Life/VisiGen platform using genetically engineered DNA polymerases along with attached fluorescent dyes to create an enhanced signal after incorporation of the nucleotide by fluorescence resonance energy transfer (FRET); and the LI-COR Biosciences' platform using dye-quencher nucleotides in sequencing reactions.

Other NGS methods include, but are not limited to, nanopore sequencing, sequencing by hybridization, nanotransistor-array-based sequencing, Polony sequencing, scanning electron tunneling microscopy (STM)-based sequencing, and nanowire molecular sensor-based sequencing.

Nanopore sequencing involves electrophoresis of nucleic acid molecules in solution through nano-scale pores that provide a highly airtight area that can be analyzed in single-nucleic-acid polymers. Representative nanopore sequencing methods are described in Branton D. et al., Nat. Biotechnol. 2008; 26(10):1146-53] and elsewhere.

Sequencing by hybridization is a non-enzymatic method using DNA microarrays. Typically, a single pool of DNA is fluorescently labeled and hybridized to an array containing a known sequence. The hybridization signal from a given spot on the array can be used to identify the DNA sequence. Binding of one strand of DNA to another strand complementary thereto in a DNA double strand is sensitive even to single-base mismatches when the hybrid region is short or when a specified mismatch detection protein is present. Representative hybridization sequencing methods are described, for example, in Hanna G. J. et al., J. Clin. Microbiol. 2000; 38(7): 2715-21; and Edwards J. R. et al., Mut. Res. 2005; 573(1-2): 3-12.

Polony sequencing is based on Polony amplification and multiple single-base-extension (FISSEQ). Polony amplification is a method of amplifying DNA *in situ* on a polyacrylamide film. Representative Polony sequencing methods are described, for example, in US Patent Application Publication No. 2007/0087362.

Nanotransistor-array-based devices such as carbon nanotube field effect transistors (CNTFETs) can also be used for NGS. For example, DNA molecules are extended and driven across nanotubes by microfabricated electrodes. DNA molecules sequentially contact the carbon nanotube surface, and the difference in current flow from the respective bases is created due to charge transfer between the DNA molecule and the nanotube. DNA is sequenced by recording the difference. Representative nanotransistor-array-based sequencing methods are described, for example, in US Patent Publication No. 2006/0246497.

Scanning tunneling microscopy (STM) can also be used for NGS. Using a piezoelectrically controlled probe that performs a raster scan of the specimen, STM forms an image on the surface thereof. STM can be used to image the physical properties of single DNA molecules, causing coherent electron tunneling imaging and spectroscopy, for example, by integrating a flexible actuator-driven gap with a scanning tunneling microscope. Representative sequencing methods using STM are described, for example, in US Patent Application Publication No. 2007/0194225.

Molecular analysis devices consisting of nanowiremolecular sensors can also be used for NGS. Such devices can detect the interaction of nitrogenous substances disposed on nucleic acid molecules and nanowires such as DNA. Molecular guides are disposed to guide molecules near the molecular sensors to allow interaction and subsequent detection. Representative sequencing methods using nanowire molecular sensors are described, for example, in US Patent Application Publication No. 2006/0275779.

Double-stranded sequencing may be used for NGS. Double-stranded sequencing uses blocking and unblocking primers to sequence both the sense and antisense strands of DNA. Typically, this method includes: annealing an unblocking primer to a first strand of a nucleic acid; annealing a second blocking primer to a second strand of the nucleic acid; extending the nucleic acid along the first strand with a polymerase; terminating the first sequencing primer; deblocking the second primer; and extending the nucleic acid along the second strand. Representative double-stranded sequencing methods are described, for example, in US Pat. No. 7,244,567.

Data analysis stage. After NGS reads are formed, they are aligned or *de novo* assembled to a known reference sequence.

For example, identification of genetic modifications such as single-nucleotide polymorphisms and structural variants in a sample (e.g., a tumor sample) can be performed by aligning NGS reads to a reference sequence (e.g., a wild-type sequence). A method of aligning NGS reads to sequences is described, for example, in Trapnell C. and Salzberg S.L. Nature Biotech., 2009, 27:455-457.

Examples of the *de novo* assembly are described, for example, in Warren R. et al., Bioinformatics, 2007, 23:500-501; Butler J. et al., Genome Res., 2008, 18:810-820; and Zerbino D.R. and Birney E., Genome Res., 2008, 18:821-829.

Sequence alignment or assembly can be performed using read data from one or more NGS platforms, for example, by mixing Roche/454 and Illumina/Solexa read data. In the present invention, the alignment is not limited thereto, but may be performed using the BWA algorithm and the hg19 sequence.

In the present invention, the step (B) of determining the position of the nucleic acid fragments may preferably be performed through sequence alignment, and the sequence alignment includes a computational method or approach using a computer algorithm to determine the case where there is a possibility that a sequence (e.g., a short-read sequence e.g., by next-generation sequencing) is derived from the genome or the case where there is an identity therebetween by evaluating the similarity between a read sequence and a reference sequence. Various algorithms may be applied to the sequence alignment problem. Some algorithms are relatively slow, but enable relatively high specificity. These include, for example, dynamic programming-based algorithms. Dynamic programming is a method of solving complicated problems by segmenting them into simpler steps. Other approaches are more efficient, but are typically not exhaustive, and include, for example, heuristic algorithms and probabilistic methods designed for massive database searches.

Typically, the alignment process may include two steps, namely candidate screening and sequence alignment. Candidate screening reduces the search space for sequence alignments from the entire genome for a shorter enumeration of possible alignment positions. As the term literally implies, sequence alignment includes aligning sequences including sequences provided during the candidate screening. This may be performed using a broad alignment (e.g., a Needleman-Wunsch alignment) or a local alignment (e.g., a Smith-Waterman alignment).

Most attribute sorting algorithms may have one of three types based on the indexing method: algorithms based on hash tables (e.g. BLAST, ELAND, SOAP), suffix trees (e.g. Bowtie, BWA), and merge sort (for example, slider). Short read sequences are typically used for alignment. Examples of sequence alignment algorithms/programs for short-read sequences include, but are not limited to, BFAST (Homer N. et al., PLoS One. 2009;4(11):e7767), BLASTN (from blast.ncbi.nlm.nih.gov on the World Wide Web), BLAT (Kent W. J. Genome Res. 2002;12(4):656-64), Bowtie (Langmead B. et al., Genome Biol. 2009;10(3):R25), BWA (Li H. and Durbin R., Bioinformatics, 2009, 25:1754-60), BWA-SW (Li H. and Durbin R., Bioinformatics, 2010;26(5):589-95), CloudBurst (Schatz M. C., Bioinformatics, 2009;25(11):1363-9), Corona Lite (Applied Biosystems, Carlsbad, California, USA), CASHX (Fahlgren N. et al., RNA, 2009; 15, 992-1002), CUDA-EC (Shi H. et al., J. Comput. Biol. 2010;17(4):603-15), ELAND (bioit.dbi.udel.edu/howto/eland on the World Wide Web), GNUMAP (Clement N. L. et al., Bioinformatics. 2010;26(1):38-45), GMAP (Wu T.D. and Watanabe C.K., Bioinformatics, 2005;21(9):1859-75), GSNAP (Wu T.D. and Nacu S., Bioinformatics, 2010;26(7):873-81), Geneious Assembler (Biomatters Ltd., Oakland, New Zealand), LAST, MAQ (Li H. et al., Genome Res. 2008;18(11):1851-8), Mega-BLAST (at ncbi.nlm.nih.gov/blast/megablast.shtml on the World Wide Web), MOM (Eaves H.L. and Gao Y. Bioinformatics. 2009;25(7):969-70), MOSAIK (at bioinformatics.bc.edu/marthlab/Mosaik on the World Wide Web), NovoAlign (at novocraft.com/main/index.php on the World Wide Web), PALMapper (at fml.tuebingen.mpg.de/raetsch/suppl/palmapper on the World Wide Web), PASS (Campagna D. et al., Bioinformatics, 2009;25(7):967-8), PatMaN (Prufer K. et al., Bioinformatics, 2008; 24(13):1530-1), PerM (Chen Y. et al., Bioinformatics, 2009, 25 (19):2514-2521), ProbeMatch (Kim Y. J. et al., Bioinformatics. 2009;25(11):1424-5), QPalma (de Bona F. et al., Bioinformatics, 2008, 24(16): i174), RazerS (Weese D. et al., Genome Research, 2009, 19:1646-1654), RMAP (Smith A.D. et al., Bioinformatics, 2009;25(21):2841-2), SeqMap (Jiang H. et al., Bioinformatics, 2008;24:2395-2396.), Shrec (Salmela L., Bioinformatics, 2010;26(10):1284-90), SHRiMP (Rumble S.M. et al., PLoS Comput. Biol., 2009, 5(5):e1000386), SLIDER (Malhis N. et al., Bioinformatics, 2009, 25 (1): 6-13), SLIM Search (Muller T. et al., Bioinformatics, 2001;17 Suppl 1:S182-9), SOAP (Li R. et al., Bioinformatics, 2008;24(5):713-4), SOAP2 (Li R. et al., Bioinformatics, 2009;25(15):1966-7), SOCS (Ondov B.D. et al., Bioinformatics, 2008; 24(23):2776-7), SSAHA (Ning Z. et al., Genome Res. 2001;11(10):1725-9), SSAHA2 (Ning Z. et al., Genome Res. 2001;11(10):1725-9), Stampy (Lunter G. and Goodson M., Genome Res. 2010, epub ahead of print), Taipan (at taipan.sourceforge.net on the World Wide Web), UGENE (at ugene.unipro.ru on the World Wide web), XpressAlign (at bcgsc.ca/platform/bioinfo/software/XpressAlign on the World Wide Web), and ZOOM (Bioinformatics Solutions Inc., Waterloo, Ontario, Canada).

A sequence alignment algorithm may be selected based on a number of factors including, for example, the sequencing technique, length of reads, number of reads, available computing resources, and sensitivity/scoring requirements. Different sequence alignment algorithms can achieve different levels of speed, alignment sensitivity, and alignment specificity. Alignment specificity refers to the percentage of target sequence residues that are correctly aligned compared to the predicted alignment, as typically shown in the submission. Alignment sensitivity also refers to the percentage of target sequence residues that are aligned, as shown in typically predicted alignments in the submission.

Alignment algorithms such as ELAND or SOAP can be used to align short reads (e.g., from Illumina/Solexa sequencers) to a reference genome when the speed is the first factor to be considered. Alignment algorithms such as BLAST or Mega-BLAST are used to determine similarity using shorter reads (e.g., Roche FLX) when specificity is considered the most important factor, although these methods are slower. Alignment algorithms such as MAQ or NovoAlign can be used for single- or paired-end data when the quality score is important and accuracy is thus essential (e.g.

in fast massive SNP searches). Alignment algorithms such as Bowtie or BWA use the Burrows-Wheeler Transform (BWT) and thus require a relatively small memory footprint. Alignment algorithms such as BFAST, PerM, SHRiMP, SOCS or ZOOM map color space reads and thus can be used along with ABI's SOLiD platform. In some applications, results from two or more sorting algorithms may be combined.

In the present invention, the length of the sequence information (reads) in step (B) is 5 to 5,000 bp, and the number of reads that are used may be 5,000 to 5 million, but the invention is not limited thereto.

In the present invention, the step of grouping the reads in step (C) may be performed based on the adapter sequences of the reads. FD may be calculated from the selected sequence information separately for nucleic acid fragments aligned in a forward direction and nucleic acid fragments aligned in a reverse direction, or together for the whole group.

In the present invention, the method may further include, prior to the step (C), separating nucleic acid fragments satisfying a mapping quality score from the aligned nucleic acid fragments.

In the present invention, the mapping quality score may vary depending on a desired criterion, but is preferably 15 to 70, more preferably 50 to 70, and most preferably 60.

In the present invention, the FD in step (D) is defined as the distance between the Representative Position of the $i^{th}$ nucleic acid fragment and the Representative Position of at least one nucleic acid fragment selected from the $i+1^{th}$ to the $n^{th}$ nucleic acid fragments among the obtained n nucleic acid fragments.

In the present invention, the FD is set as one or more values selected from the group consisting of sum, difference, product, mean, log of product, log of sum, median, quantile, minimum, maximum, variance, standard deviation, median absolute deviation and coefficient of variance of the distance between the Representative Position of the $1^{st}$ nucleic acid fragment and the Representative Position of at least one nucleic acid fragment selected from the group consisting of the $2^{nd}$ to $n^{th}$ nucleic acid fragments, among the obtained n nucleic acid fragments, and/or one or more reciprocals thereof, values calculated in consideration of weights, and statistical values, but the present invention is not limited thereto.

As used herein, the expression "one or more values... and/or one or more reciprocals thereof" is intended to mean that one of the numerical values described above or a combination of two or more thereof may be used.

As used herein, the expression "Representative Position of the nucleic acid fragment" may be a value obtained by adding an arbitrary value to the median of the nucleic acid fragments or subtracting the arbitrary value therefrom.

The FD for the obtained n nucleic acid fragments may be defined as follows.

$$FD = Dist(Ri \sim Rj) \quad (1 < i < j < n),$$

wherein the Dist function calculates one or more values selected from the group consisting of sum, difference, product, mean, log of product, log of sum, median, quantile, minimum, maximum, variance, standard deviation, median absolute deviation and coefficient of variance of the differences between the alignment position values of all nucleic acid fragments included between the two nucleic acid fragments Ri and Rj, and/or one or more reciprocals thereof, values calculated in consideration of weights, and statistical values, which are not limited thereto.

That is, as used herein, the FD (fragment distance) refers to the distance between aligned nucleic acid fragments. Here, the number of cases where nucleic acid fragments are selected for distance calculation may be defined as follows. When a total of N nucleic acid fragments are present, the number of combinations of distances between nucleic acid fragments is $\sum_{k=1}^{n-1} k$. That is, when i is 1, i+1 is 2 and the distance from one or more nucleic acid fragments selected from the $2^{nd}$ to $n^{th}$ nucleic acid fragments is defined.

In the present invention, the FD may be obtained by calculating the distance between a specific position inside the $i^{th}$ nucleic acid fragment and a specific position inside at least one of the $i+1^{th}$ to $n^{th}$ nucleic acid fragments.

For example, if a nucleic acid fragment has a length of 50 bp and is aligned at position 4,183 on chromosome 1, the genetic position values that can be used to calculate the distance of this nucleic acid fragment are 4,183 and 4,232 on chromosome 1.

If a nucleic acid fragment having a length of 50 bp adjacent to the nucleic acid fragment is aligned at position 4,232 of chromosome 1, the genetic position values that can be used to calculate the distance of this nucleic acid fragment are 4,232 and 4,281 of chromosome 1, and the FD between the two nucleic acid fragments is 1 to 99.

If another adjacent 50 bp nucleic acid fragment is aligned at position 4123 of chromosome 1, the genetic position values that can be used to calculate the distance of this nucleic acid fragment are 4,123 and 4,172 of chromosome 1, the FD between the two nucleic acid fragments is 61 to 159, and the FD between the nucleic acid fragment and the first exemplary nucleic acid fragment is 12 to 110, the FD may be one or more selected from the group consisting of sum, difference, product, mean, log of product, log of sum, median, quantile, minimum, maximum, variance, standard deviation, median absolute deviation and coefficient of variance of one within the range between the two FD values, and/or one or more reciprocals thereof, values calculated in consideration of weights, and statistical values, but are not limited thereto, and is preferably the reciprocal of one within the range of the two FD values, but is not limited thereto.

Preferably, in the present invention, the FD may be a value obtained by adding an arbitrary value to the median of the nucleic acid fragment or subtracting the arbitrary value therefrom.

In the present invention, the median of FD means the most centrally located value when the calculated FDs are arranged in order of size. For example, when there are three values, namely 1, 2 and 100, 2, which is central, is the median. If there is an even number of FDs, the median is determined as the mean of the two middle values. For example, if there are FDs of 1, 10, 90, and 200, the median is 50, which is the mean of 10 and 90.

In the present invention, the arbitrary value can be used without limitation, as long as it can be used to indicate the position of the nucleic acid fragment, but is preferably 0 to 5 kbp or 0 to 300% of the length of the nucleic acid fragment, 0 to 3 kbp or 0 to 200% of the length of the nucleic acid fragment, 0 to 1 kbp or 0 to 100% of the length of the nucleic acid fragment, more preferably 0 to 500 bp or 0 to 50% of the length of the nucleic acid fragment, but is not limited thereto.

In the present invention, in paired-end sequencing, the FD may be derived based on position values of forward and reverse reads.

For example, if, in a pair of 50 bp-long paired-end reads, the forward read is aligned at position 4183 of chromosome 1, and the reverse read is aligned at position 4349, both ends of this nucleic acid fragment are at positions 4183 and 4349, and Representative Positions that can be used to calculate the nucleic acid fragment distance are 4183 and 4349. At this time, if, in another paired-end read pair adjacent to the nucleic acid fragment, the forward read is aligned at position 4349 of chromosome 1, and the reverse read is aligned at position 4515, the position values of the nucleic acid fragment are 4349 and 4515. The distance between the two nucleic acid fragments may be 0 to 333, and most preferably may be 166, which is the distance corresponding to the median of the respective nucleic acid fragments.

In the present invention, when sequence information is obtained by the paired-end sequencing, the method may further include excluding nucleic acid fragments having a mapping quality score below a cutoff value from the calculation process.

In the present invention, in single-end sequencing, the FD may be derived based on one type of position value of forward or reverse read.

In the present invention, in the single-end sequencing, if a position value is derived based on sequence information read aligned in the forward direction, an arbitrary value is added thereto, and if a position value is derived based on sequence information read aligned in the reverse direction, an arbitrary value is subtracted. The arbitrary value may be used without limitation, as long as the FD clearly indicates the position of the nucleic acid fragment, but is preferably 0 to 5 kbp or 0 to 300% of the length of the nucleic acid fragment, 0 to 3 kbp or 0 to 200% of the length of the nucleic acid fragment, 0 to 1 kbp or 0 to 100% of the length of the nucleic acid fragment, more preferably 0 to 500 bp or 0 to 50% of the length of the nucleic acid fragment, but is not limited thereto.

Nucleic acids to be analyzed in the present invention may be sequenced and expressed in units called "reads". The reads may be divided into single-end sequencing reads (SE) and paired-end sequencing reads (PE) depending on the sequencing method. SE-type read means a read obtained by sequencing one of a 5' and 3' end of a nucleic acid molecule to a predetermined length in a random direction, and PE-type read means a read obtained by sequencing both 5' and 3' ends of a nucleic acid molecule to a predetermined length. It is well known to those skilled in the art that due to this difference, one read is generated from one nucleic acid fragment when sequencing in the SE mode, whereas a pair of two reads is generated from one nucleic acid fragment in the PE mode.

The ideal method to accurately calculate the distance between nucleic acid fragments includes sequencing nucleic acid molecules from the beginning to the end, aligning the reads, and using the median (center) of the position values of the aligned reads. However, the method faces technical restrictions due to limitations on sequencing technology and cost aspects. Therefore, sequencing is performed using a method such as SE or PE. In the PE mode, the start and end positions of the nucleic acid molecule can be recognized, so the exact position (median) of the nucleic acid fragment can be determined through the combination of these values. In the SE mode, only information on one end of the nucleic acid fragment can be used, so there is a limitation on accuracy of calculation of the position (median).

Also, when calculating the distance between nucleic acid molecules using the end information of all reads sequenced (aligned) in both forward and reverse directions, a value may be inaccurately calculated due to the factor of the sequencing direction.

Therefore, for technical reasons related to the sequencing method, the 5' end of the forward read has a small position value and the 3' end of the reverse read has a large position value, compared to the central position value of the nucleic acid molecule. When an arbitrary value (extended bp) is added to the forward read and subtracted from the reverse read, using this feature, a value close to the central position of the nucleic acid molecule can be estimated.

That is, the arbitrary value (extended bp) may vary depending on the sample that is used, and cell-free nucleic acids are known to have an average nucleic acid length of about 166 bp, and thus the arbitrary value (extended bp) thereof is set to about 80 bp. If the experiment is performed using fragmentation (e.g. sonication) equipment, about half of the target length set during the fragmentation process may be set as extended bp.

In the present invention, step (E) of determining the chromosomal abnormality includes:

(E-i) determining a representative FD (RepFD) for the entire chromosomal region or for each specific region;

(E-ii) calculating one or more selected from the group consisting of sum, difference, product, mean, log of product, log of sum, median, quantile, minimum, maximum, variance, standard deviation, median absolute deviation and coefficient of variance of RepFD in a certain region in a sample, other than the entire chromosomal region or specific genomic region, and/or the reciprocals thereof, to derive a normalized factor;

(E-iii) calculating a representative FD ratio (RepFD ratio) based on Equation 1 below; and

$$\text{Equation 1: RepFD ratio = RepFD Target genomic region / Normalized Factor}$$

(E-iv) comparing the RepFD ratio between a normal reference group and the sample to calculate a fragment distance index (FDI).

In the present invention, the representative FD (RepFD) of step (E-i) is at least one selected from the group consisting of a sum, difference, product, mean, log of product, log of sum, median, quantile, minimum, maximum, variance, standard deviation, median absolute deviation and coefficient of variance of FD and/or the reciprocals thereof, and is preferably a median, mean or reciprocal of FDs, but is not limited thereto.

In the present invention, the entire chromosomal region or specific genomic region may be used without limitation, as long as it is a set of human nucleic acid sequences, but may preferably be a chromosomal unit or a specific region of some chromosomes. For example, a specific region for detecting numerical abnormalities may be an autosome, which is considered to be euploid, and a specific region for detecting structural abnormalities may be any genomic region excluding regions (centromere, telomere) having low inherency, but is not limited thereto.

The certain region in the sample other than the entire chromosomal region or specific genomic region to be analyzed in step (E-ii) is selected using a method including:

a) randomly selecting a region other than an entire chromosomal region or a specific genomic region to be analyzed;

b) determining a representative RepFD of the genomic region selected in step a) with a pre-normalized factor (PNF) ;

c) calculating a representative FD ratio (RepFD ratio) based on Equation 2:

$$\text{Equation 2: RepFD ratio = RepFD Target genomic region / PNF}$$

d) calculating a coefficient of variance (SD/ mean) of the RepFD ratio of a normal reference group; and

e) determining a genomic region having a smallest value among coefficients of variance obtained by repeatedly performing steps a) to d) as the certain region in the sample other than the entire chromosomal region or the specific genomic region.

In the present invention, step e) may be repeated 100 or more times, preferably between 10,000 and 1 million times, and most preferably 100,000 times, but the invention is not limited thereto.

In the present invention, step (E-iv) may include comparing the RepFD ratio of the normal reference group with the RepFD ratio of the sample.

In the present invention, any method of comparing the RepFD ratio of the normal reference group with the RepFD ratio of the sample may be used without limitation, as long as it is capable of identifying whether the two values are statistically significantly different, but is preferably a mean/standard-deviation-based Z-score, a median-based log ratio, a likelihood ratio calculated using other classification algorithms, or the like, most preferably a Z-score based on mean and standard deviation, but is not limited thereto.

In the present invention, the fragment distance index is calculated by comparing the RepFD ratio of the normal reference group with the RepFD ratio of the sample to be analyzed. A standard score method such as a Z score may be used for comparison, and the threshold is an integer or range such as an infinite positive or negative number, and is preferably 3, but is not limited thereto.

In another aspect, the present invention is directed to a device for determining a chromosomal abnormality, the device including:

a decoder configured to extract nucleic acids from a biological sample and decode sequence information;

an aligner configured to align the decoded sequence to a reference genome database; and

a chromosomal abnormality determiner configured to measure the distance between the aligned nucleic acid fragments among the selected nucleic acid fragments to thereby calculate a fragment distance (FD), to calculate a fragment distance index (FDI) over the entire chromosomal region or each specific genomic region based on the calculated FD, and to determine that there is a chromosomal abnormality when the FDI does not fall within a cutoff value range.

In another aspect, the present invention is directed to a computer-readable storage medium including an instruction configured to be executed by a processor for detecting a chromosomal abnormality through the following steps including:

(A) extracting nucleic acids from a biological sample and obtaining nucleic acid fragments to obtain sequence information therefrom;

(B) aligning the nucleic acid fragments to a reference genome database based on the obtained sequence information (reads);

(C) measuring the distance between the selected nucleic acid fragments to calculate a fragment distance (FD); and

(D) calculating a fragment distance index (FDI) over the entire chromosomal region or in each specific genomic region based on the FD calculated in step (C) and determining that there is a chromosomal abnormality when the FDI does not fall within a cutoff value range.

Specifically, the computer-readable storage medium includes an instruction configured to be executed by a processor for detecting a chromosomal abnormality through the following steps:

(A) extracting nucleic acids from a biological sample and obtaining nucleic acid fragments to obtain sequence information therefrom;

(B) identifying the positions of the nucleic acid fragments in a reference genome database based on the obtained sequence information (reads);

(C) grouping the aligned nucleic acid fragments into whole sequences, forward sequences and reverse sequences based on the obtained sequence information (reads);

(D) measuring the distance between the Representative Positions of the aligned nucleic acid fragments for the grouped nucleic acid fragments, to calculate a fragment distance (FD) for each group; and

(E) calculating a fragment distance index (FDI) for the entire chromosomal region or each specific region based on the FD for each group calculated in step (D) and determining that there is a chromosomal abnormality when the FDI does not fall within a cutoff value range.

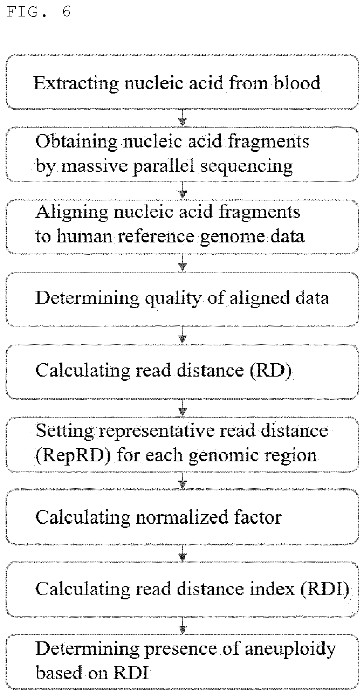

In another embodiment of the present invention, it was confirmed that the distance between the reads can be calculated by calculating the position values of both ends of the reads by adding 50% of the average length of the nucleic acid fragment to be analyzed to the median of the aligned nucleic acid fragments or subtracting 50% of the average length therefrom (FIG. 6).

In another aspect, the present invention is directed to a method for detecting a chromosomal abnormality, the method including:

(A) extracting nucleic acids from a biological sample to obtain sequence information;

(B) aligning the obtained sequence information (reads) to a reference genome database;

(C) measuring the distance between the aligned reads in the aligned sequence information (reads) to calculate a read distance (RD); and

(D) calculating a read distance index (RDI) over the entire chromosomal region or in each specific genomic region based on the RD calculated in step (C) and determining that there is a chromosomal abnormality when the RDI does not fall within a cutoff value range.

In the present invention,
step A) includes:

(A-i) obtaining nucleic acids from blood, semen, vaginal cells, hair, saliva, urine, oral cells, amniotic fluid containing placental cells or fetal cells, tissue cells, and a mixture thereof;

(A-ii) removing proteins, fats, and other residues from the collected nucleic acids using a salting-out method, a column chromatography method, or a bead method to obtain purified nucleic acids;

(A-iii) producing a single-end sequencing or pairend sequencing library for the purified nucleic acids or nucleic acids randomly fragmented by an enzymatic digestion, pulverization, or hydroshear method;

(A-iv) reacting the produced library with a next-generation sequencer; and

(A-v) obtaining sequence information (reads) of the nucleic acids in the next-generation sequencer.

In the present invention, the length of the sequence information (reads) in step (B) is 5 to 5000 bp, and the number of reads that are used may be 5000 to 5 million, but the invention is not limited thereto.

In the present invention, step (c) may further include grouping the aligned reads depending on the alignment direction.

In the step of grouping the reads, the grouping may be performed based on the adapter sequences of the aligned reads. RD may be calculated from the selected sequence information separately for reads aligned in a forward direction and reads aligned in a reverse direction.

In the present invention, the method may further include, prior to the step (C), separating reads satisfying a mapping quality score from the aligned reads.

In the present invention, the mapping quality score may vary depending on a desired criterion, but is preferably 15 to 70, more preferably 50 to 70, and most preferably 60.

In the present invention, the RD in step (C) may be calculated based on the distance from the value of one of both ends of the $i^{th}$ read and one or more reads selected from the $i+1^{th}$ to the $n^{th}$ reads, with respect to the obtained n reads, to the value obtained by adding 50% of the average length of the nucleic acid thereto or subtracting 50% of the average length therefrom.

In the present invention, the RD may be selected from the group consisting of sum, difference, product, mean, log of product, log of sum, median, quantile, minimum, maximum, variance, standard deviation, median absolute deviation, coefficient of variance, reciprocals thereof and combinations thereof, values calculated in consideration of weights, and statistical values, but is not limited thereto.

In the present invention, the median of RD means the most centrally located value when the calculated RDs are arranged in order of size. For example, when there is an odd number of values, such as 1, 2 and 100, 2, which is the most central, is the median. If there is an even number of RDs, the median is determined as the mean of the two middle values. For example, if there are RDs of 1, 10, 90, and 200, the median is 50, which is the mean of 10 and 90.

In the present invention, the RD is obtained by calculating the distance between the 5' or 3' end inside the $i^{th}$ read and the 5' or 3' end inside at least one of the $i+1^{th}$ to $n^{th}$ reads.

For example, if, in a pair of 50 bp-long paired-end reads, the forward read is aligned at position 4183 of chromosome 1, and the reverse read is aligned at position 4349, both ends of this nucleic acid fragment are at positions 4183 and 4349, and the Representative Positions that can be used to calculate the nucleic acid fragment distance are 4183 and 4349. At this time, if, in another paired-end read pair adjacent to the nucleic acid fragment, the forward read is aligned at position 4349 of chromosome 1 and the reverse read is aligned at position 4515, the position values of the nucleic acid fragment are 4349 and 4515. The distance between the two nucleic acid fragments may be 0 to 333, and most preferably may be 166, which is the distance between the medians of the respective nucleic acid fragments. In the above example, when the average length of the nucleic acid fragment is 166, and 50% of the average length of the nucleic acid fragment is subtracted from the median (4266), the position value of the first nucleic acid fragment is 4183, the position value of the second nucleic acid fragment is 4349, and the distance between the reads is 166 (from 4349 to 4183). Meanwhile, when 50% is added to the median, the position value of the first nucleic acid fragment is 4349, the position value of the second nucleic acid fragment is 4515, and the distance between the reads is 166 (from 4515 to 4349).

In the present invention, step (D) of determining the chromosomal abnormality includes:

(D-i) determining a representative RD (RepRD) for each entire chromosomal region or specific genomic region;

(D-ii) calculating one or more selected from the group consisting of a sum, difference, product, mean, log of product, log of sum, median, quantile, minimum, maximum, variance, standard deviation, median absolute deviation, and coefficient of variance of RepRD in a certain region in the sample other than the entire chromosomal region or specific genomic region, and/or the reciprocals thereof, to derive a normalized factor;

(D-iii) calculating a representative RD ratio (RepRD ratio) based on Equation 10 below; and

$$\text{Equation 10: RepRD ratio = RepRD Target genomic region / Normalized Factor}$$

(D-iv) comparing the RepRD ratio of a normal reference group with that of the sample to calculate a read distance index (RDI).

In the present invention, the representative RD (RepRD) of step (D-i) is one or more selected from the group consisting of a sum, difference, product, mean, log of product, log of sum, median, quantile, minimum, maximum, variance, standard deviation, median absolute deviation, and coefficient of variance of RD, and/or the reciprocal thereof, and a statistic value, but is not limited thereto, and is preferably a median, mean or reciprocal of RDs, but is not limited thereto.

In the present invention, "median of RepRD" means the most centrally located value when the calculated RepRD values are arranged in order of size. For example, when there is an odd number of values such as 1, 2 and 100, 2, which is the most central, is the median. If there is an even number of RepRD values, the median is determined as the mean of the two middle values. For example, if there are RepRD values of 1, 10, 90, and 200, the median is 50, which is the mean of 10 and 90.

In the present invention, the entire chromosome region or specific genomic region may be used without limitation as long as it is a population of human nucleic acid sequences, but may preferably be a chromosomal unit or a specific region of some chromosomes. For example, a specific region for detecting numerical abnormalities may be an autosome that is considered to be euploid, and a specific region for detecting structural abnormalities may be any genomic region excluding regions (centromere, telomere) having low inherency, but is not limited thereto.

The certain region in the sample other than the entire chromosome region or specific genomic region to be analyzed in step (D-ii) is selected using a method including:

a) randomly selecting a region other than an entire chromosome region or a specific genomic region to be analyzed;

b) determining a representative RepRD of the genomic region selected in step a) with a pre-normalized factor (PNF) ;

c) calculating a representative RD ratio (RepRD ratio) based on Equation 11:

$$\text{Equation 11: RepRD ratio = RepRD Target genomic region / PNF}$$

d) calculating a coefficient of variance (SD/mean) of the RepRD ratio of a normal reference group; and

e) determining a genomic region having a smallest value among coefficients of variance obtained by repeatedly performing steps a) to d) as the certain region in the sample other than the entire chromosome region or the specific genomic region.

In the present invention, step e) may be repeated 100 or more times, preferably between 10,000 and 1 million times, and most preferably 100,000 times, but the invention is not limited thereto.

In the present invention, step (iv) may include comparing the RepRD ratio of the normal reference group with the RepRD ratio of the sample.

In the present invention, any method of comparing the RepRD ratio of the normal reference group with the RepRD ratio of the sample may be used without limitation, as long as it is capable of identifying the fact that the two values are statistically significantly different, but is preferably a mean/standard-deviation-based Z-score, a median-based log ratio, a likelihood ratio calculated through another classification algorithm, or the like, most preferably a Z-score based on mean and standard deviation, but is not limited thereto.

In the present invention, the read distance index is calculated by comparing the Rep RD ratio of the normal reference group with the Rep RD ratio of the sample to be analyzed. A standard score method such as a Z score may be used for comparison, and the threshold is an integer or range such as an infinite positive or negative number, and is preferably -3 or 3, but is not limited thereto.

In another aspect, the present invention is directed to a device for determining a chromosomal abnormality, including: a decoder configured to extract nucleic acids from a biological sample and decode sequence information;

an aligner configured to align the decoded sequence to a reference genome database; and

a chromosomal abnormality determiner, configured to measure the distance between the aligned reads in the selected sequence information (reads) to thereby calculate a read distance (RD), to calculate a read distance index (RDI) over the entire chromosome region or each specific genomic region based on the calculated RD, and to determine that there is a chromosomal abnormality when the RDI does not fall within a cutoff value range.

In another aspect, the present invention is directed to a computer-readable storage medium including an instruction configured to be executed by a processor for detecting a chromosomal abnormality, through the following steps including:

(A) extracting nucleic acids from a biological sample to obtain sequence information;

(B) aligning the obtained sequence information (reads) to a reference genome database;

(C) measuring the distance between the aligned reads in the selected sequence information (reads) to calculate a read distance (RD); and

(D) calculating a read distance index (RDI) over the entire chromosome region or in each specific genomic region based on the RD calculated in step (C) and determining that there is a chromosomal abnormality when the RDI does not fall within a cutoff value range.

**Example**

Hereinafter, the present invention will be described in more detail with reference to examples. However, it will be obvious to those skilled in the art that these examples are provided only for illustration of the present invention, and should not be construed as limiting the scope of the present invention.

**Example 1. Extracting DNA from blood to perform next-generation sequencing**

10 mL of a sample was collected from the blood and stored in an EDTA tube. Within 2 hours after blood collection, only the plasma was primarily centrifuged at 1,200g and 4°C for 15 minutes, and then the primarily centrifuged plasma was secondarily centrifuged at 16,000g and 4°C for 10 minutes to isolate the plasma supernatant excluding the precipitate. Cell-free DNA was extracted from the isolated plasma using a Tiangenmicro DNA kit (Tiangen). Paired-end (PE) data was generated using a DNBseq G400 (MGI) (50 cycles * 2) after performing a library preparation process using an MGIEasy cell-free DNA Library Prep set kit (MGI), and single-end (SE) data was generated using a Nextseq500 (Illumina) after performing a library preparation process using a TruSeq Nano DNA HT library prep kit (Illumina).

Sequence information of about 10 million nucleic acid fragments could be obtained from PE data, and sequence information of about 1.3 million nucleic acid fragments could be obtained from SE data.

**Example 2. Quality control of sequence information data and FD calculation**

The base sequence information was pre-processed, and the following series of processes was performed before calculating the FD value. The library sequences were aligned based on the Hg19 sequence of the reference chromosome using the BWA-mem algorithm in the fastq format file generated by the next-generation sequencer (NGS). There is a possibility that an error may occur when aligning the library sequences, so two steps were performed to correct the error. First, the duplicated library sequences were removed and then sequences having a mapping quality score lower than 60 were removed from the library sequences aligned by a BWA-mem algorithm.

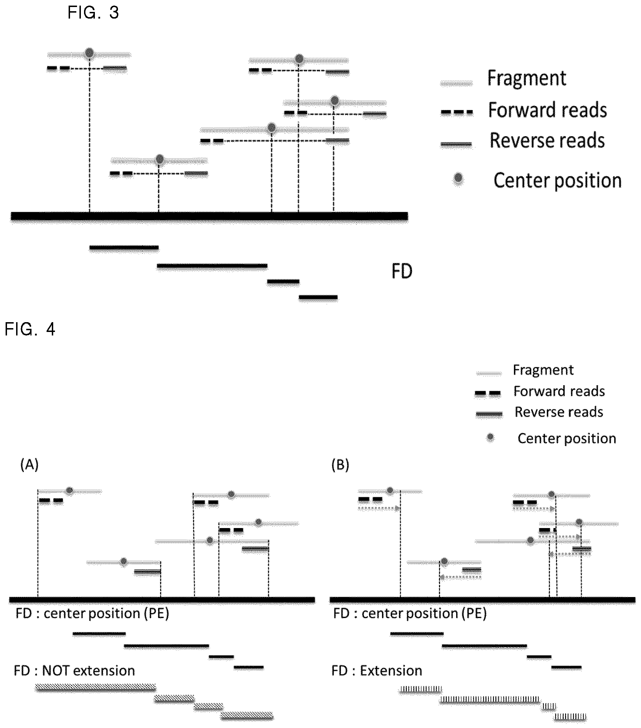

The selected reads were grouped into forward and reverse reads depending on the alignment direction, the distance between adjacent reads was calculated as an FD using Equation 3 below, and the concept thereof is shown in FIGS. 2 and 3. The D function of Equation 3 below is a function to calculate the difference between the genomic position values. a and b in Equation 3 below are the position values of the nucleic acid fragments, may be either the minimum or the maximum among the position values of two pieces of aligned sequence information for PE sequencing, and may be a position value of the aligned sequence information or a value obtained by extending a specific value to the position value for SE sequencing.

$$\text{Equation 3: } Fragment\ Distance\ (FD)\ =\ D(a,b)\ |\ a\ \in$$

$$Fi\ ,\ b\ \in\ Fi)$$

**Example 3. Confirmation of difference in FD according to extension**

PE data provides information about the positions of the beginning and end of the nucleic acid fragments, and the distance between the nucleic acid fragments can be calculated based on the central position. PE data are randomly grouped into "For" and "Rev" reads, FD for reads classified as "For" is calculated based on the 5' position of the "For" reads, FD for reads classified as "Rev" is calculated based on the 3' position of the "Rev" reads, and extension was performed by adding 80 bp to the For reads and subtracting 80 bp from the Rev reads.

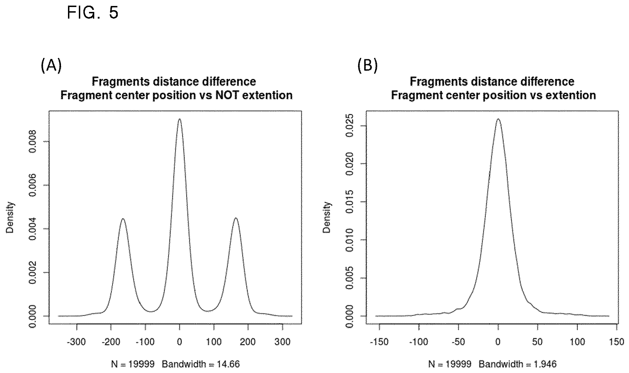

The difference between the FD in the process described above and the FD after the extension process was compared. It can be seen from the result shown in FIG. 5 that the FD calculated after extension is similar to the centered FD of PE and the FD calculated before extension is different at +166 and -166.

**Example 4. FDI calculation**

4-1. FDI calculation for detecting chromosomal numerical abnormalities

The FDI was calculated using the SE sequencing data, and the extension value was set to 80 bp. For the chromosomes to be detected for aneuploidy, the median ratio of RepFD of the selected chromosome set was defined as a normalized factor, and was calculated using Equation 4 below.

[282]     [Table 1]

CHROMOSOMES

TO BE

DETECTED                              CHROMOSOME SET

FOR

ANEUPLOIDY

| | |
|---|---|
| **13** | 1,2,3,4,5,6,7,8,9,10,11,12,14,15,16,17,19,20,22 |
| **18** | 1,2,3,4,5,6,7,8,9,10,11,12,14,15,16,17,19,20,22 |
| **21** | 1,2,3,4,5,6,7,8,9,10,11,12,14,15,16,17,19,20,22 |

Equation 4: $Normalized\ Factor = Median\ of\ RepFD_{selected\ chromosome\ set}$

wherein selected chromosome set corresponds to a population of chromosomes in Table 1 above.

The RepFD ratio was calculated in Equation 5 below using the FD and normalized factor calculated in Equations 3 and 4, respectively.

Equation 5: $RepFD\ ratio = RepFD_{Target\ chromosome} / Normalized\ Factor$

The mean and standard deviation of the RepFD ratio were calculated in a reference group including 2000 normal subjects, and the fragment distance index (FDI) of the sample to be analyzed was calculated using Equation 6.

Equation 6: $FDI = (MEAN(RepFD\ Ratio_{reference} - RepFD\ Ratio_{sample} -) / SD(RepFD\ Ratio_{reference})$

All nucleic acid fragments were applied to Equation 6 (Equation 7), nucleic acid fragments aligned in the forward direction were applied thereto (Equation 8), and nucleic acid fragments aligned in the reverse direction were applied thereto (Equation 9).

Equation 7: $FDI^{all} = mean(RepFD^{all}\ Ratio_{reference}) - RepFD^{all}\ Ratio_{sample} / SD(RepFD^{all}\ Ratio_{reference})$

$$\text{Equation 8: } FDI^{For} = \text{mean}(RepFD^{For} \text{ Ratio}_{reference}) - RepFD^{For}$$

$$\text{Ratio}_{sample} / SD(RepFD^{For} \text{ Ratio}_{reference})$$

$$\text{Equation 9: } FDI^{Rev} = \text{mean}(RepFD^{Rev} \text{ Ratio}_{reference}) - RepFD^{Rev}$$

$$\text{Ratio}_{sample} / SD(RepFD^{Rev} \text{ Ratio}_{reference})$$

4-2. Confirmation of performance of FDI for detecting chromosomal numerical abnormalities

The result of analysis on 2000 samples from the normal standard group and 88 clinical samples including 6 trisomy samples showed 100% sensitivity and 100% specificity.

The threshold value for positive confirmation was 3 for all of FD121$^{all}$, FDI21$^{For}$, and FDI21$^{Rev}$.

For each sample, three FDI values were calculated for positive confirmation, and the case where all of them were 3 or more was finally determined to be positive. Three samples (G19NIPT261-3, G19NIPT261-10, G19NIPT261-13) among the 88 samples analyzed was finally determined to be negative because one FDI was determined to be positive, but the remaining two FDI were determined to be negative.

[Table 2]

| sample | Result | FDI21.All | RDI21.For | FDI21.rev |
|---|---|---|---|---|
| G19NIPT264-22 | T21 | 13.5189 | 18.9706 | 22.7449 |
| G19NIPT261-27 | T21 | 11.3797 | 15.7714 | 16.3134 |
| G19NIPT262-11 | T21 | 9.8365 | 13.4254 | 12.6415 |
| G19NIPT263-21 | T21 | 9.6024 | 13.3521 | 13.8184 |
| G19NIPT264-29 | T21 | 9.3665 | 12.3367 | 14.4694 |
| G19NIPT261-3 | T21 | 6.2652 | 7.9757 | 8.6841 |
| G19NIPT263-12 | N | 2.8936 | 3.5516 | 1.5855 |
| G19NIPT263-10 | N | 1.9594 | 2.6681 | 3.1888 |
| G19NIPT263-13 | N | 1.7782 | 2.0879 | 3.8235 |

Example 5. Extracting DNA from blood for analysis based on RD and performing next-generation sequencing

10 mL of blood was collected from 400 normal subjects, 175 subjects with trisomy 21, 67 subjects with trisomy 18, and 26 subjects with trisomy 13, and was stored in an EDTA tube. Within 2 hours after blood collection, only the plasma was primarily centrifuged at 1,200g and 4°C for 15 minutes, and then the primarily centrifuged plasma was secondarily centrifuged at 16,000g and 4°C for 10 minutes to isolate the plasma supernatant excluding the precipitate. Cell-free DNA was extracted from the isolated plasma using a Tiangenmicro DNA kit (Tiangen), a library was prepared using an MGIEasy cell-free DNA Library Prep set kit (MGI), and sequencing was performed in a 75 single-end mode using a Nextseq500 (Illumina).

As a result, about 13 million reads per sample were produced.

**Example 6. Quality control of sequence information data and RD calculation**

The base sequence information was pre-processed and the following series of processes was performed before calculating the RD value. The library sequences were aligned based on the Hg19 sequence of the reference chromosome using the BWA-mem algorithm in the fastq format file generated by the next-generation sequencer (NGS). There is a possibility that an error may occur when aligning the library sequences, so two steps were performed to correct the error. First, the duplicated library sequences were removed, and then sequences having a mapping quality score lower than 60 were removed from among the library sequences aligned by a BWA-mem algorithm.

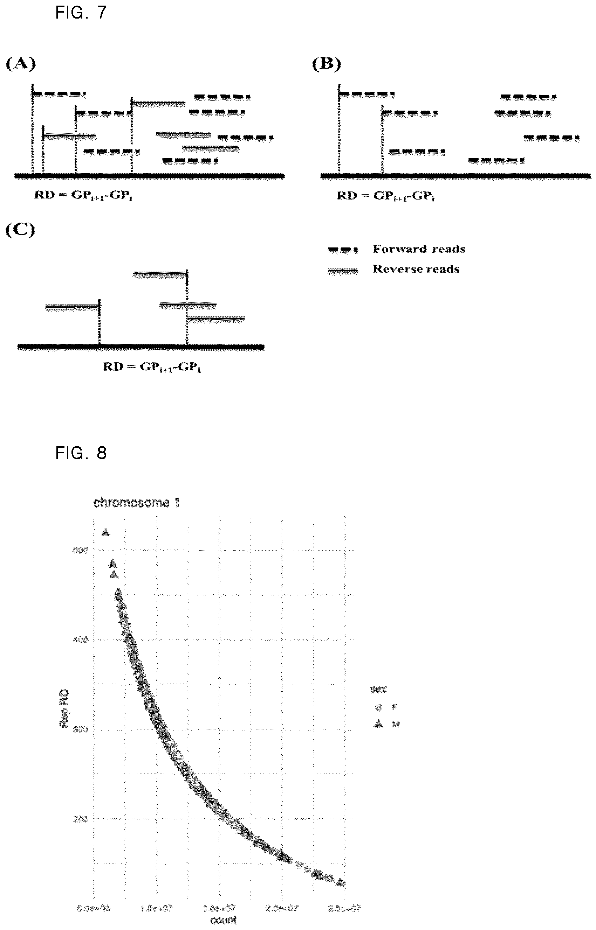

The selected reads were grouped into forward and reverse reads depending on the alignment direction, the distance between adjacent reads was calculated as an RD using Equation 12 below, and the concept thereof is shown in FIG. 7. The D function of Equation 12 below is a function to calculate the difference between the genomic position values.

$$\text{Equation 12: } Read\ Distance\ (RD) = D(a,b)\ |\ a \in Ri\ ,\ b \in Ri)$$

**Example 7. RDI calculation**

7-1. RDI calculation for detecting chromosomal numerical abnormalities

The median of RD was defined as RepRD for each chromosome. For the chromosomes to be detected for aneuploidy, the median ratio of RepRD of the selected chromosome set was defined as a normalized factor, and was calculated by Equation 13 below.

[Table 3]

| CHROMOSOMES TO BE DETECTED FOR ANEUPLOIDY | CHROMOSOME SET |
|---|---|
| 13 | 4,6 |
| 18 | 5,8 |
| 21 | 2,4,14,20 |

$$\text{Equation 13: } Normalized\ Factor = Median\ of\ RepRD_{selected\ chromosome\ set}$$

wherein the selected chromosome set corresponds to a population of chromosomes shown in Table 3 above.

The RepRD ratio was calculated in Equation 14 below using the RD and normalized factor calculated in Equations 12 and 13, respectively.

$$\text{Equation 14: } RepRD\ ratio = RepRD_{Target\ chromosome} / Normalized\ Factor$$

The mean and standard deviation of the RepRD ratio were calculated in a reference group including 400 normal subjects, and the read distance index (RDI) of the sample to be analyzed was calculated using Equation 15.

$$\text{Equation 15: } RDI = RepRD\ Ratio_{sample} - MEAN(RepRD\ Ratio_{reference}) / SD(RepRD\ Ratio_{reference})$$

7-2. RDI calculation for chromosomal structural abnormalities

The chromosome was uniformly cleaved into segments 50k bases long, and then the median of RD for each segment was defined as RepRD. Also, the median of autosomal RD was used as a normalized factor. Data from 437 normal women were used for the reference group, and the mean and standard deviation of the RepRD ratio were calculated for each segment of the chromosome. The RDI was calculated using Equation 15.

7-3. Confirmation of performance depending on representative RD (RepRD) calculation method (using reciprocal of median)

The RD values of the sequence information aligned in each genetic region (for each chromosome) were calculated, and the reciprocal of the median of RD values was defined as a representative RD (RepRD). Here, the median means the most centrally located value when the calculated RDs are arranged in order of size. For example, given the three values 1, 2 and 100, 2, which is central, is the median.

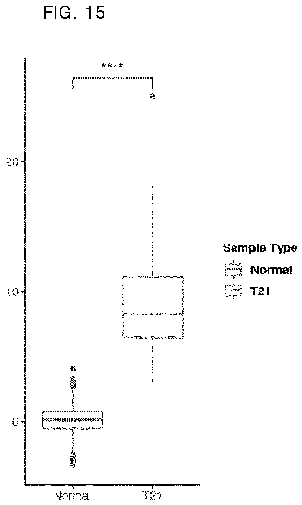

If there is an even number of RDs, the median is determined as the mean of the two middle values. For example, if there are RD values of 1, 10, 90, and 200, the median is 50, which is the mean of 10 and 90, which are located in the center. 49 samples confirmed as trisomy 21 and 3,448 samples confirmed as normal were used as analysis samples, and the RepRD was the reciprocal of the median of the RDs. In the analysis method, the RDI was calculated using the Z-score method using the mean and standard deviation of the RepRD of 3,448 normal samples. As the result of analysis, whether or not the sample had an abnormality with regard to the number of chromosomes could be detected with an accuracy of about 0.999 (Table 4, FIG. 15).

[Table 4]

| Chromosome | Accuracy (95%CI) | Sensitivity | Specificity | AUC |
|---|---|---|---|---|
| T21 | 0.9994(0.9979, 0.9999) | 0.9795 | 0.9997 | 1.0000 |

7-4. Confirmation of performance depending on representative RD (RepRD) calculation method (using mean)

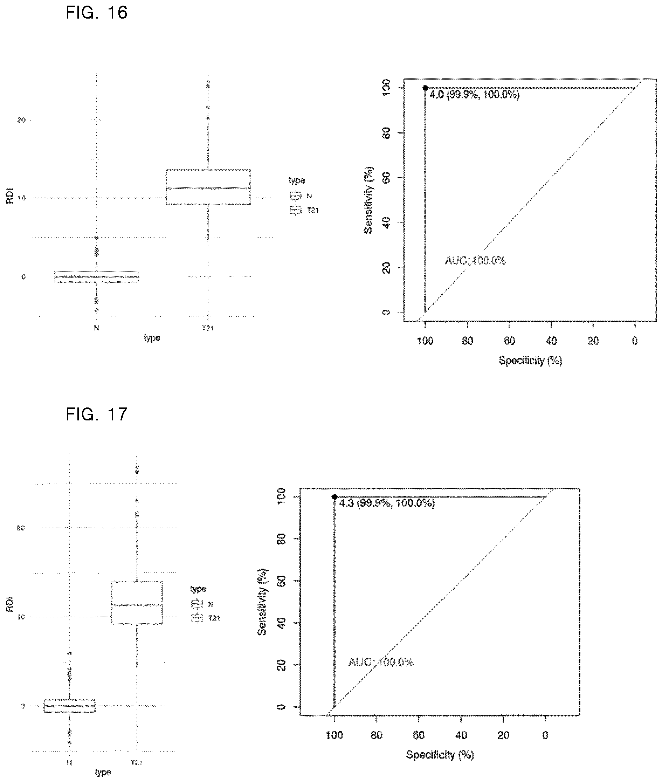

The RDs of sequence information aligned in each genetic region (for each chromosome) were calculated, and the mean of RDs was defined as a representative RD (RepRD). Here, the mean is the arithmetic average of the calculated RDs. If there are RDs of 10, 50, and 90, 50, which is (10+50+90)/3, is the representative RD. The RDI was calculated using the Z-score method using the RepRD mean and standard deviation of a normal group using 1999 normal subjects and 163 T21 samples. The chromosomes used as the normalized factor were 2, 7, 9, 12, and 14. As a result of the analysis, an abnormality in the number of chromosomes in the sample could be detected with an accuracy of about 0.9995, and when the threshold was set to 4.0, it was confirmed that the sensitivity was 0.999 and the specificity was 1.000 (Table 5, FIG. 16) .

[Table 5]

| Chromosome | Accuracy | Sensitivity | Specificity | AUC |
|---|---|---|---|---|
| T21 | 0.9995(0.9974,1.0000) | 0.9999 | 1.0000 | 1.0000 |

7-5. Confirmation of performance depending on representative RD (RepRD) calculation method (using reciprocal of mean)

The RDs of sequence information aligned in each genetic region (for each chromosome) were calculated, and the mean of RDs was defined as a representative RD (RepRD). Here, the mean is the arithmetic average of the calculated RDs. If there are RDs of 10, 50 and 90, 50, which is (10+50+90)/3, is the mean, and the reciprocal of this value, 1/50 = 0.02, was used as a representative RD value. The RDI was calculated using the Z-score method using the RepRD mean and standard deviation of a normal group with respect to 1,999 normal subjects and 163 T21 samples. The chromosomes used as the normalized factor were 2, 7, 8, 9, 12, and 14. As a result of the analysis, an abnormality in the number of chromosomes in the sample could be detected with an accuracy of about 0.9995, and when the threshold was set to 4.3, it was confirmed that the sensitivity was 0.993 and that the specificity was 1.000 (Table 6, FIG. 17).

[Table 6]

| Chromosome | Accuracy | Sensitivity | Specificity | AUC |
|---|---|---|---|---|
| T21 | 0.9995(0.9974,1.0000) | 0.9939 | 1.000 | 1.000 |

**Example 8. Confirmation of performance of RDI for detecting chromosomal numerical abnormalities**

8-1. Distribution of Read Count and Read Distance

In an analysis using the concept of the distance of aligned reads, as the number of reads that are generated increases, the distance between the reads decreases. To verify this, the distribution of the number of reads and the distribution of RepRD for each chromosome were analyzed.

The result showed that the RepRD decreased as the overall number of reads increased. In particular, it was confirmed that the relationship between the number of reads and the RepRD was not linear but non-linear, which is a result indicating that the distance between the reads reflects the positions of the reads as well as the number of reads (FIG. 8).

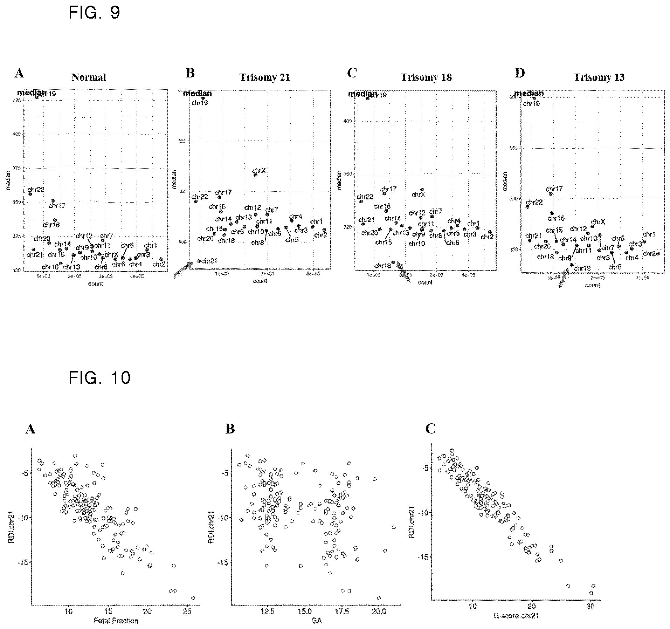

It was confirmed that the distribution of the RepRD of the chromosomes identified as aneuploidy in trisomy 13, 18 and 21 samples was low compared to the normal sample (FIG. 9) .

8-2. Relationship between performance of read distance index, fetal fraction, clinical information, and conventional G-score

In a fetal aneuploidy test using maternal blood, the fetal fraction and pregnancy weeks greatly affect the accuracy of the test. As the pregnancy week increases, the fetal fraction increases, and as the fetal fraction increases, the accuracy of the test increases. The result of analysis of the $RDI_{chr21}$ of the trisomy 21 sample, the maternal pregnancy week, and the distribution of fetal fraction showed that the $RDI_{chr21}$ decreased as the fetal fraction increased. Also, with respect to the relationship between the pregnancy week and the $RDI_{chr21}$, the $RDI_{chr21}$ decreases in the sample of 15 weeks or longer, and the conventional G-score based on read count had a tendency similar thereto (Korean Patent No. 10-1686146 (FIG. 10).

8-3. Result of positive confirmation clinical sample analysis

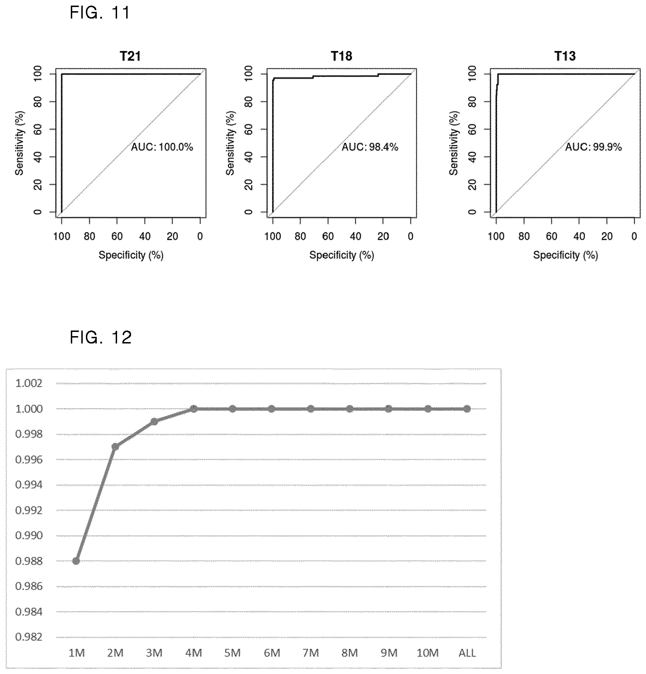

The analysis performance of the normal group and the samples identified as having chromosomal aneuploidy was verified using the RDI. The RDI was set as a predetermined cutoff of -3, and the analytical performance was compared between normal and aneuploid samples. The result showed that the accuracy for trisomy 13 was 0.991, the accuracy for trisomy 18 was 0.989, and the accuracy for trisomy 21 was 0.998 (Table 7). In addition, it was confirmed that the AUCs were 0.999, 0.984, and 1.000 for trisomy 13, 18, and 21, respectively (FIG. 11).

[Table 7]

| Chromosome | Accuracy (95%CI) | Sensitivity | Specificity | AUC |
|---|---|---|---|---|
| T13 | 0.991(0.976-0.997) | 0.846 | 1.000 | 0.999 |
| T18 | 0.989(0.975-0.997) | 0.925 | 1.000 | 0.984 |
| T21 | 0.998(0.99-1) | 1.000 | 0.998 | 1.000 |

8-4. Confirmation of performance depending on RDI calculation method

The result of log ratio analysis using the median, which is different from the Z-score method using the mean and standard deviation of the RDI ratio of the normal reference group, was confirmed. Equation 16 was used for the log ratio analysis method.

$$\text{Equation 16: } RDI = log_{10}(RepRD\ Ratio_{sample}\ /\ Median(RepRD\ Ratio_{reference}))$$

The same sample that was used in Example 8-3 was used, the RDI was set as a predetermined cutoff (-0.0045), and analysis performance was compared. The performance depended slightly on the positive type and the accuracy was 0.976 for trisomy 21, 0.994 for trisomy 18, and 0.991 for trisomy 13 (Table 8).

[Table 8]

| Chromosome | Accuracy (95%CI) | Sensitivity | Specificity | AUC |
|---|---|---|---|---|
| T13 | 0.991(0.976-0.997) | 0.846 | 1.000 | 0.999 |
| T18 | 0.994(0.981-0.999) | 0.955 | 1.000 | 0.984 |
| T21 | 0.976(0.959-0.987) | 1.000 | 0.965 | 1.000 |

8-5. Confirmation of down-sampling performance

The amount of data (number of reads) produced using a non-invasive method for determining the presence of fetal aneuploidy using next-generation sequencing is known to be an important factor for accuracy. In this embodiment, the analysis performance of the RDI method depending on the number of reads was calculated. The criterion for analysis performance was determined using the AUC of the ROC analysis, and the number of reads was determined using in-silico random read selection. One to ten million reads were randomly selected. The result of analysis using the No. 21 aneuploidy sample showed that analysis performance decreased as the number of reads decreased (FIG. 12).

**Example 9. Confirmation of performance of RDI for detecting chromosomal structural abnormalities**

9-1. Distribution of read count and read distance

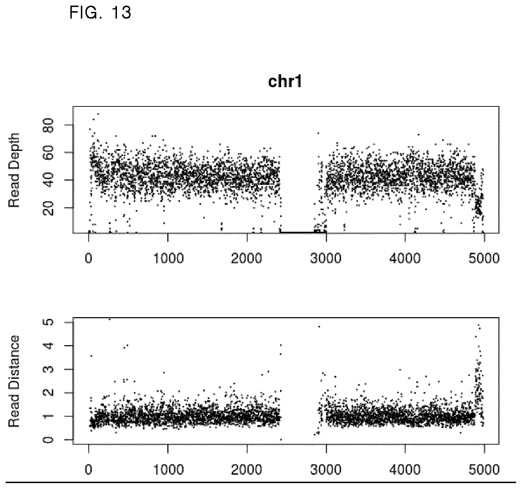

In order to determine whether or not there is a chromosomal structural abnormality using RDI, it is necessary to divide the chromosome into an appropriate size, and in this embodiment, the chromosome is segmented into a size of 50k bases. The distance between the reads decreases as the number of reads increases, and the distance between the reads increases as the number of reads increases. The relationship between the number of reads in each section and the distance between the reads was determined. The result showed that the distance between the reads in the region where a structural abnormality of the chromosome was detected was longer than that of the region where no structural abnormality of the chromosome was detected (FIG. 13).

9-2. Comparison with result of microarray

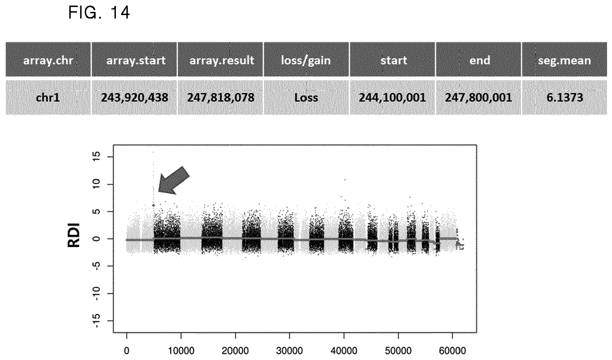

The results of microarray and RDI analysis, which detect chromosomal structural abnormalities, were compared. The analysis sample was a sample in which a deletion of 3,897,640 bp in length was detected at the end of chromosome 1, and the result of analysis using RDI showed that a structural abnormality (deletion) having a size of 3,700,000 bp was detected in a region similar thereto (FIG. 14).

Although specific configurations of the present invention have been described in detail, those skilled in the art will appreciate that this description is provided to set forth preferred embodiments for illustrative purposes, and should not be construed as limiting the scope of the present invention. Therefore, the substantial scope of the present invention is defined by the accompanying claims and equivalents thereto.

[Industrial applicability]

The method of determining a chromosomal abnormality according to the present invention includes grouping aligned nucleic acid fragments and then using the distance between the Representative Positions of the nucleic acid fragments, unlike conventional methods that determine the amount of chromosomes based on the read count. The conventional method decreases in accuracy when the number of reads decreases, whereas the method of the present invention is useful because detection accuracy is increased even when the number of reads decreases, and detection accuracy is high when the distance between nucleic acid fragments of a certain region, rather than all chromosome regions, is analyzed.

**Claims**

1. A method of detecting a chromosomal abnormality, the method comprises: calculating a distance between Representative Positions of nucleic acid fragments extracted from a biological sample.

2. The method according to claim 1, wherein the nucleic acid fragments are cell-free nucleic acids or intracellular nucleic acids.

3. The method according to claim 1, wherein the nucleic acid fragments are obtained by direct sequencing, next-generation sequencing, or sequencing through nonspecific whole-genome amplification.

4. The method according to claim 3, comprising:

   (A) extracting nucleic acids from a biological sample and obtaining nucleic acid fragments to obtain sequence information therefrom;
   (B) identifying positions of the nucleic acid fragments in a reference genome database based on the obtained sequence information (reads);
   (C) grouping the sequence information (reads) into whole sequences, forward sequences, and reverse sequences;
   (D) defining Representative Positions of the respective nucleic acid fragments using the grouped sequence information, and measuring the distance between the Representative Positions to calculate a fragment distance (FD) for each group; and
   (E) calculating a fragment distance index (FDI) for the entire chromosomal region or each specific region based on the FD for each group calculated in step (D) and determining that there is a chromosomal abnormality when the FDI does not fall within a cutoff value range.

5. The method according to claim 4, wherein the step (A) comprises:

   (A-i) obtaining nucleic acids from blood, semen, vaginal cells, hair, saliva, urine, oral cells, amniotic fluid containing placental cells or fetal cells, tissue cells, and a mixture thereof;
   (A-ii) removing proteins, fats, and other residues from the collected nucleic acids using a salting-out method, a column chromatography method, or a bead method to obtain purified nucleic acids;
   (A-iii) producing a single-end sequencing or pair-end sequencing library for the purified nucleic acids or nucleic acids randomly fragmented by an enzymatic digestion, pulverization, or hydroshear method;
   (A-iv) reacting the produced library with a next-generation sequencer; and
   (A-v) obtaining sequence information (reads) of the nucleic acids in the next-generation sequencer.

6. The method according to claim 4, wherein the FD in step (D) is calculated as a distance between the Representative Position of an $i^{th}$ nucleic acid fragment and the Representative Position of at least one nucleic acid fragment selected from $i+1^{th}$ to $n^{th}$ nucleic acid fragments among obtained n nucleic acid fragments.

7. The method according to claim 6, wherein the Representative Position of the nucleic acid fragment is obtained by adding an arbitrary value to a median of the nucleic acid fragment or subtracting the arbitrary value therefrom.

8. The method according to claim 7, wherein, in paired-end sequencing, the Representative Position of the nucleic acid fragment is derived based on position values of forward and reverse reads.

9. The method according to claim 8, further comprising excluding nucleic acid fragments having a mapping quality score of reads below a cutoff value from calculation.

10. The method according to claim 6, wherein in single-end sequencing, the Representative Position of the nucleic acid fragment is derived based on one type of position value of forward or reverse read.

11. The method according to claim 10, wherein an arbitrary value is added when a position value is derived based on sequence information aligned in the forward direction and the arbitrary value is subtracted when a position value is derived based on sequence information aligned in the reverse direction.

12. The method according to claim 7, wherein the arbitrary value is 30 to 70% of a mean length of the nucleic acid to be analyzed.

13. The method according to claim 7, wherein the arbitrary value is 0 to 5 kbp or 0 to 300% of a length of the nucleic acid fragment.

14. The method according to claim 4, wherein step (E) comprises:

(E-i) determining a representative FD (RepFD) for an entire chromosomal region or for each specific region;
(E-ii) calculating one or more selected from the group consisting of a sum, difference, product, mean, log of product, log of sum, median, quantile, minimum, maximum, variance, standard deviation, median absolute deviation and coefficient of variance of RepFD, reciprocals thereof and a combination thereof in a certain region in the sample, rather than the entire chromosomal region or specific genomic region, to derive a normalized factor;
(E-iii) calculating a representative FD ratio (RepFD ratio) based on Equation 1 below; and

$$\text{Equation 1: RepFD ratio = RepFD Target genomic region / Normalized Factor}$$

(E-iv) comparing the RepFD ratio of a normal reference group with that of the sample to calculate a fragment distance index (FDI).

15. The method according to claim 14, wherein the representative FD (RepFD) of step (E-i) is at least one selected from the group consisting of a sum, difference, product, mean, log of product, log of sum, median, quantile, minimum, maximum, variance, standard deviation, median absolute deviation and coefficient of variance of FDs, and/or a reciprocal thereof.

16. The method according to claim 15, wherein the representative FD (RepFD) of step (E-i) is a median, mean, or reciprocal of FDs.

17. The method according to claim 14, wherein the certain region in the sample other than the entire chromosomal region or specific genomic region in step (E-ii) is selected using a method comprising:

a) randomly selecting a region other than an entire chromosomal region or a specific genomic region to be analyzed;
b) determining a representative RepFD of the genomic region selected in step a) with a pre-normalized factor (PNF) ;
c) calculating a representative FD ratio (RepFD ratio) based on Equation 2:

$$\text{Equation 2: RepFD ratio = RepFD Target genomic region / PNF}$$

d) calculating a coefficient of variance (SD/mean) of the RepFD ratio of a normal reference group; and
e) determining a genomic region having a smallest value among obtained coefficients of variance obtained by repeatedly performing steps a) to d) as the certain region in the sample, other than the entire chromosomal region or the specific genomic region.

18. The method according to claim 14, wherein step (E-iv) comprises comparing the RepFD ratio of the normal reference group with the RepFD ratio of the sample.

19. A device for determining a chromosomal abnormality, the device comprising:

a decoder configured to extract nucleic acids from a biological sample and decode sequence information;
an aligner configured to align the decoded sequence to a reference genome database; and
a chromosomal abnormality determiner configured to measure the distance between the aligned nucleic acid fragments among the selected nucleic acid fragments to thereby calculate a fragment distance (FD), to calculate a fragment distance index (FDI) over the entire chromosomal region or each specific genomic region based on the calculated FD, and to determine that there is a chromosomal abnormality when the FDI does not fall within a cutoff value range.

20. A computer-readable storage medium including an instruction configured to be executed by a processor for detecting a chromosomal abnormality through the following steps comprising:

(A) extracting nucleic acids from a biological sample and obtaining nucleic acid fragments to obtain sequence information;
(B) aligning the nucleic acid fragments to a reference genome database based on the obtained sequence information (reads);
(C) measuring the distance between the selected nucleic acid fragments to calculate a fragment distance (FD); and
(D) calculating a fragment distance index (FDI) over the entire chromosomal region or in each specific genomic region based on the FD calculated in step (C) and determining that there is a chromosomal abnormality when the FDI does not fall within a cutoff value range.

21. The method according to claim 7, wherein the calculated FD is a read distance (RD) when the arbitrary value is 50% of a mean length of the nucleic acid to be analyzed.

22. A method for detecting a chromosomal abnormality, the method comprising:

(A) extracting nucleic acids from a biological sample to obtain sequence information;
(B) aligning the obtained sequence information (reads) to a reference genome database;
(C) measuring a distance between the aligned reads in the aligned sequence information (reads) to calculate a read distance (RD); and
(D) calculating a read distance index (RDI) over an entire chromosomal region or in each specific genomic region based on the RD calculated in step (C), and determining that there is a chromosomal abnormality when the RDI does not fall within a cutoff value range.

23. The method according to claim 22, wherein step (A) is performed using a method comprising:

(A-i) obtaining nucleic acids from blood, semen, vaginal cells, hair, saliva, urine, oral cells, amniotic fluid containing placental cells or fetal cells, tissue cells, and a mixture thereof;
(A-ii) removing proteins, fats, and other residues from the collected nucleic acids using a salting-out method, a column chromatography method, or a bead method to obtain purified nucleic acids;
(A-iii) producing a single-end sequencing or pair-end sequencing library for the purified nucleic acids or nucleic acids randomly fragmented by an enzymatic digestion, pulverization, or hydroshear method;
(A-iv) reacting the produced library with a next-generation sequencer; and
(A-v) obtaining sequence information (reads) of the nucleic acids in the next-generation sequencer.

24. The method according to claim 22, further comprising, prior to the step (C), grouping the aligned reads according to an alignment direction.

25. The method according to claim 22, wherein the RD in step (C) is calculated by a distance from a value of one of both ends of an $i^{th}$ read and one or more reads selected from $i+1^{th}$ to $n^{th}$ reads, among obtained n reads, to the value obtained by adding 50% of an average length of the nucleic acid thereto or subtracting 50% of the average length of the nucleic acid therefrom.

26. The method according to claim 22, wherein the RD is obtained by calculating the distance between the 5' or 3' end inside the $i^{th}$ read and the 5' or 3' end inside at least one of the $i+1^{th}$ to $n^{th}$ reads.

27. The method according to claim 22, wherein the step (D) comprises:

(D-i) determining a representative RD (RepRD) for each entire chromosomal region or specific genomic region;
(D-ii) calculating one or more selected from the group consisting of a sum, difference, product, mean, log of product, log of sum, median, quantile, minimum, maximum, variance, standard deviation, median absolute deviation and coefficient of variance of RepRD in a certain region in the sample other than the entire chromosomal region or specific genomic region, and/or a reciprocal thereof, to derive a normalized factor;
(D-iii) calculating a representative RD ratio (RepRD ratio) based on Equation 10 below;

$$\text{Equation 10: RepRD ratio = RepRD Target genomic region / Normalized Factor;}$$

and
(D-iv) comparing the RepRD ratio of a normal reference group with that of the sample to calculate a read distance index (RDI).

28. The method according to claim 27, wherein the representative RD (RepRD) of step (D-i) is one or more selected from the group consisting of a sum, difference, product, mean, log of product, log of sum, median, quantile, minimum, maximum, variance, standard deviation, median absolute deviation and coefficient of variance of RD, and/or reciprocals thereof.

29. The method according to claim 28, wherein the representative RD (RepRD) of step (D-i) is a median, mean, or reciprocal of RDs.

30. The method according to claim 27, wherein the certain region in the sample other than the entire chromosome region or specific genomic region to be analyzed in step (D-ii) is selected using a method comprising:

   a) randomly selecting a region other than the entire chromosome or specific genomic region to be analyzed;
   b) determining a representative RepRD of the genomic region selected in step a) with a pre-normalized factor (PNF) ;
   c) calculating a representative RD ratio (RepRD ratio) based on Equation 11:

$$\text{Equation 11: RepRD ratio = RepRD Target genomic region / PNF;}$$

   d) calculating a coefficient of variance (SD/mean) of the RepRD ratio of a normal reference group; and
   e) determining a genomic region having a smallest value among coefficients of variance obtained by repeatedly performing steps a) to d) as the certain region in the sample other than the entire chromosome region or the specific genomic region.

31. The method according to claim 27, wherein step (D-iv) comprises comparing the RepRD ratio of the normal reference group with the RepRD ratio of the sample.

32. A device for determining a chromosomal abnormality, the device comprising:

   a decoder configured to extract nucleic acids from a biological sample and decode sequence information;
   an aligner configured to align the decoded sequence to a reference genome database; and
   a chromosomal abnormality determiner configured to measure the distance between the aligned reads among the selected sequence information (reads) to calculate a read distance (RD), to calculate a read distance index (RDI) over the entire chromosomal region or each specific genomic region based on the calculated RD, and to determine that there is a chromosomal abnormality when the RDI does not fall within a cutoff value range.

33. A computer-readable storage medium including instructions configured to be executed by a processor for detecting a chromosomal abnormality through the following steps comprising:

   (A) extracting nucleic acids from a biological sample to obtain sequence information;
   (B) aligning the obtained sequence information (reads) to a reference genome database;
   (C) measuring the distance between the aligned reads among the selected sequence information (reads) to calculate a read distance (RD); and
   (D) calculating a read distance index (RDI) over the entire chromosomal region or in each specific genomic region based on the RD calculated in step (C) and determining that there is a chromosomal abnormality when the RDI does not fall within a cutoff value range.

FIG. 1

Extracting nucleic acid
from blood

Obtaining nucleic acid fragments
by massive parallel sequencing

Aligning nucleic acid fragments
to human reference genome data

Determining quality of aligned data

Forward reads

Calculating fragment distance (FD)

Calculating representative fragment
distance (Rep FD)

Calculating normalized factor

Calculating fragment distance index
(FDI)

All reads

Calculating fragment distance (FD)

Calculating representative fragment
distance (Rep FD)

Calculating normalized factor

Calculating fragment distance index
(FDI)

Reverse reads

Calculating fragment distance (FD)

Calculating representative fragment
distance (Rep FD)

Calculating normalized factor

Calculating fragment distance index
(FDI)

Determining presence of aneuploidy based
on three FDIs

FIG. 2

FIG. 3

| | |
|---|---|
| ⎯⎯ | Fragment |
| ▬ ▬ ▬ | Forward reads |
| ═══ | Reverse reads |
| ● | Center position |

FD

FIG. 4

| | |
|---|---|
| ⎯⎯ | Fragment |
| ▬ ▬ | Forward reads |
| ═══ | Reverse reads |
| ● | Center position |

(A)

FD : center position (PE)

FD : NOT extension

(B)

FD : center position (PE)

FD : Extension

FIG. 5

(A)

**Fragments distance difference**
**Fragment center position vs NOT extention**

N = 19999 Bandwidth = 14.66

(B)

**Fragments distance difference**
**Fragment center position vs extention**

N = 19999 Bandwidth = 1.946

FIG. 6

```
┌─────────────────────────────────────┐
│   Extracting nucleic acid from blood │
└─────────────────────────────────────┘
                  │
                  ▼
┌─────────────────────────────────────┐
│   Obtaining nucleic acid fragments   │
│   by massive parallel sequencing     │
└─────────────────────────────────────┘
                  │
                  ▼
┌─────────────────────────────────────┐
│   Aligning nucleic acid fragments    │
│   to human reference genome data     │
└─────────────────────────────────────┘
                  │
                  ▼
┌─────────────────────────────────────┐
│   Determining quality of aligned data│
└─────────────────────────────────────┘
                  │
                  ▼
┌─────────────────────────────────────┐
│   Calculating read distance (RD)     │
└─────────────────────────────────────┘
                  │
                  ▼
┌─────────────────────────────────────┐
│   Setting representative read distance│
│   (RepRD) for each genomic region    │
└─────────────────────────────────────┘
                  │
                  ▼
┌─────────────────────────────────────┐
│   Calculating normalized factor      │
└─────────────────────────────────────┘
                  │
                  ▼
┌─────────────────────────────────────┐
│   Calculating read distance index (RDI)│
└─────────────────────────────────────┘
                  │
                  ▼
┌─────────────────────────────────────┐
│   Determining presence of aneuploidy │
│   based on RDI                       │
└─────────────────────────────────────┘
```

FIG. 7

(A)

RD = GP$_{i+1}$-GP$_i$

(B)

RD = GP$_{i+1}$-GP$_i$

(C)

RD = GP$_{i+1}$-GP$_i$

- - - Forward reads
— Reverse reads

FIG. 8

chromosome 1

FIG. 9

FIG. 10

FIG. 11

FIG. 12

FIG. 13

chr1

FIG. 14

| array.chr | array.start | array.result | loss/gain | start | end | seg.mean |
|---|---|---|---|---|---|---|
| chr1 | 243,920,438 | 247,818,078 | Loss | 244,100,001 | 247,800,001 | 6.1373 |

FIG. 15

FIG. 16

FIG. 17

## INTERNATIONAL SEARCH REPORT

| International application No. |
|---|
| **PCT/KR2020/010853** |

| A. | CLASSIFICATION OF SUBJECT MATTER |
|---|---|

**G16B 20/20**(2019.01)i; **G16B 15/00**(2019.01)i; **G16B 30/10**(2019.01)i; **G16B 40/20**(2019.01)i

According to International Patent Classification (IPC) or to both national classification and IPC

| B. | FIELDS SEARCHED |
|---|---|

Minimum documentation searched (classification system followed by classification symbols)

G16B 20/20; C12Q 1/68; C12Q 1/6806; C12Q 1/6827; C12Q 1/6869; G06F 19/18; G16B 15/00; G16B 30/10; G16B 40/20

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 핵산(nucleic acid), 단편(fragment), 거리(distance), 염색체이상(chromosome aberration)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | KR 10-2019-0041510 A (RESOLUTION BIOSCIENCE, INC.) 22 April 2019. See claims 1-59. | 1-33 |
| A | KR 10-2015-0082228 A (THE CHINESE UNIVERSITY OF HONG KONG) 15 July 2015. See entire document. | 1-33 |
| A | JP 2017-073144 A (SEQUENOM, INC.) 13 April 2017. See entire document. | 1-33 |
| A | KR 10-2018-0009763 A (ZYTOVISION GMBH) 29 January 2018. See entire document. | 1-33 |
| A | KR 10-2018-0031742 A (THE CHINESE UNIVERSITY OF HONG KONG) 28 March 2018. See entire document. | 1-33 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **27 November 2020** | **27 November 2020** |

| Name and mailing address of the ISA/KR | Authorized officer |
|---|---|
| **Korean Intellectual Property Office**<br>**Government Complex Daejeon Building 4, 189, Cheongsa-ro, Seo-gu, Daejeon, Republic of Korea**<br>**35208** | |
| Facsimile No. **+82-42-481-8578** | Telephone No. |

Form PCT/ISA/210 (second sheet) (July 2019)

# EP 4 020 484 A1

**INTERNATIONAL SEARCH REPORT**
Information on patent family members

International application No.

**PCT/KR2020/010853**

| Patent document cited in search report | | | Publication date (day/month/year) | Patent family member(s) | | | Publication date (day/month/year) |
|---|---|---|---|---|---|---|---|
| KR | 10-2019-0041510 | A | 22 April 2019 | CN | 109804080 | A | 24 May 2019 |
| | | | | EP | 3504347 | A1 | 03 July 2019 |
| | | | | JP | 2019-526257 | A | 19 September 2019 |
| | | | | US | 2018-0163272 | A1 | 14 June 2018 |
| | | | | WO | 2018-039463 | A1 | 01 March 2018 |
| KR | 10-2015-0082228 | A | 15 July 2015 | CN | 104781422 | A | 15 July 2015 |
| | | | | EP | 2898100 | A1 | 29 July 2015 |
| | | | | EP | 2898100 | B1 | 22 November 2017 |
| | | | | EP | 3354747 | A1 | 01 August 2018 |
| | | | | EP | 3536807 | A1 | 11 September 2019 |
| | | | | JP | 6317354 | B2 | 25 April 2018 |
| | | | | JP | 6683752 | B2 | 22 April 2020 |
| | | | | JP | 2015-536639 | A | 24 December 2015 |
| | | | | JP | 2018-121644 | A | 09 August 2018 |
| | | | | JP | 2020-108400 | A | 16 July 2020 |
| | | | | KR | 10-2148547 | B1 | 26 August 2020 |
| | | | | KR | 10-2020-0085928 | A | 15 July 2020 |
| | | | | WO | 2014-043763 | A1 | 27 March 2014 |
| JP | 2017-073144 | A | 13 April 2017 | EP | 2764459 | A2 | 13 August 2014 |
| | | | | JP | 6073902 | B2 | 01 February 2017 |
| | | | | JP | 6227095 | B2 | 08 November 2017 |
| | | | | JP | 2014-534507 | A | 18 December 2014 |
| | | | | JP | 2017-099419 | A | 08 June 2017 |
| | | | | WO | 2013-052913 | A2 | 11 April 2013 |
| | | | | WO | 2013-052913 | A3 | 07 November 2013 |
| | | | | WO | 2013-052913 | A4 | 27 December 2013 |
| KR | 10-2018-0009763 | A | 29 January 2018 | CN | 107810276 | A | 16 March 2018 |
| | | | | CN | 108467884 | A | 31 August 2018 |
| | | | | EP | 3109324 | B1 | 19 September 2018 |
| | | | | EP | 3314010 | A1 | 02 May 2018 |
| | | | | EP | 3314010 | B1 | 09 September 2020 |
| | | | | JP | 6630823 | B2 | 15 January 2020 |
| | | | | JP | 2018-517436 | A | 05 July 2018 |
| | | | | JP | 2019-213537 | A | 19 December 2019 |
| | | | | KR | 10-2019-0116548 | A | 14 October 2019 |
| | | | | US | 2018-0282795 | A1 | 04 October 2018 |
| | | | | WO | 2016-207325 | A1 | 29 December 2016 |
| KR | 10-2018-0031742 | A | 28 March 2018 | AU | 2018-295616 | A1 | 15 February 2018 |
| | | | | CA | 2993362 | A1 | 26 January 2017 |
| | | | | CN | 108026572 | A | 11 May 2018 |
| | | | | EP | 3325664 | A1 | 30 May 2018 |
| | | | | EP | 3325664 | A4 | 02 January 2019 |
| | | | | JP | 2018-524991 | A | 06 September 2018 |
| | | | | TW | 201718872 | A | 01 June 2017 |
| | | | | US | 2017-0024513 | A1 | 26 January 2017 |
| | | | | WO | 2017-012592 | A1 | 26 January 2017 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 101686146
- US 20070087362
- US 20060246497 A
- US 20070194225
- US 20060275779
- US 7244567 B

### Non-patent literature cited in the description

- **PARK, H., KIM et al.** *Nat Genet,* 2010, vol. 42, 400-405
- **KIDD, J.M. et al.** *Nature,* 2008, vol. 453, 56-64
- **WEAVER, S. et al.** *Methods,* 2010, vol. 50, 271-276
- **DEEPAK, S. et al.** *Curr Genomics,* 2007, vol. 8, 234-251
- **MUJEZINOVIC F. et al.** *Obstet. Gynecol.,* 2007, vol. 110 (3), 687-94
- **LO Y. M. et al.** *The Lancet,* 1997, vol. 350 (9076), 485-7
- **GIL MM et al.** *Ultrasound Obstet. Gynecol.,* 2015, vol. 45 (3), 249-66
- **METZKER, M.** *Nature Biotechnology Reviews,* 2010, vol. 11, 31-46
- **LASKEN R. S.** *Curr Opin Microbiol,* 2007, vol. 10 (5), 510-6
- **TEWHEY R. et al.** *Nature Biotech,* 2009, vol. 27, 1025-1031
- **PORRECA GJ et al.** *Nature Methods,* 2007, vol. 4, 931-936
- **KRISHNAKUMAR S. et al.** *Proc. Natl. Acad. Sci. USA,* 2008, vol. 105, 9296-9310
- **TURNER EH et al.** *Nature Methods,* 2009, vol. 6, 315-316
- **GNIRKE A. et al.** *Nat. Biotechnol.,* 2009, vol. 27 (2), 182-9
- **BRANTON D. et al.** *Nat. Biotechnol.,* 2008, vol. 26 (10), 1146-53
- **HANNA G. J. et al.** *J. Clin. Microbiol.,* 2000, vol. 38 (7), 2715-21
- **EDWARDS J. R. et al.** *Mut. Res.,* 2005, vol. 573 (1-2), 3-12
- **TRAPNELL C. ; SALZBERG S.L.** *Nature Biotech.,* 2009, vol. 27, 455-457
- **WARREN R. et al.** *Bioinformatics,* 2007, vol. 23, 500-501
- **BUTLER J. et al.** *Genome Res.,* 2008, vol. 18, 810-820
- **ZERBINO D.R. ; BIRNEY E.** *Genome Res.,* 2008, vol. 18, 821-829
- **HOMER N. et al.** *PLoS One,* 2009, vol. 4 (11), e7767
- **KENT W. J.** *Genome Res,* 2002, vol. 12 (4), 656-64
- **LANGMEAD B. et al.** *Genome Biol,* 2009, vol. 10 (3), R25
- **LI H. ; DURBIN R.** *Bioinformatics,* 2009, vol. 25, 1754-60
- **LI H. ; DURBIN R.** *Bioinformatics,* 2010, vol. 26 (5), 589-95
- **SCHATZ M. C.** *Bioinformatics,* 2009, vol. 25 (11), 1363-9
- **FAHLGREN N. et al.** *RNA,* 2009, vol. 15, 992-1002
- **SHI H. et al.** *J. Comput. Biol.,* 2010, vol. 17 (4), 603-15
- **CLEMENT N. L. et al.** *Bioinformatics,* 2010, vol. 26 (1), 38-45
- **WU T.D. ; WATANABE C.K.** *Bioinformatics,* 2005, vol. 21 (9), 1859-75
- **WU T.D. ; NACU S.** *Bioinformatics,* 2010, vol. 26 (7), 873-81
- **LI H. et al.** *Genome Res,* 2008, vol. 18 (11), 1851-8
- **EAVES H.L. ; GAO Y.** *Bioinformatics,* 2009, vol. 25 (7), 969-70
- **CAMPAGNA D. et al.** *Bioinformatics,* 2009, vol. 25 (7), 967-8
- **PRUFER K. et al.** *Bioinformatics,* 2008, vol. 24 (13), 1530-1
- **CHEN Y. et al.** *Bioinformatics,* 2009, vol. 25 (19), 2514-2521
- **KIM Y. J. et al.** *Bioinformatics,* 2009, vol. 25 (11), 1424-5
- **DE BONA F. et al.** *Bioinformatics,* 2008, vol. 24 (16), i174
- **WEESE D. et al.** *Genome Research,* 2009, vol. 19, 1646-1654
- **SMITH A.D. et al.** *Bioinformatics,* 2009, vol. 25 (21), 2841-2
- **JIANG H. et al.** *Bioinformatics,* 2008, vol. 24, 2395-2396

- **SALMELA L.** *Bioinformatics,* 2010, vol. 26 (10), 1284-90
- **RUMBLE S.M. et al.** *PLoS Comput. Biol.,* 2009, vol. 5 (5), e1000386
- **MALHIS N. et al.** *Bioinformatics,* 2009, vol. 25 (1), 6-13
- **MULLER T. et al.** *Bioinformatics,* 2001, vol. 17 (1), 182-9
- **LI R. et al.** *Bioinformatics,* 2008, vol. 24 (5), 713-4
- **LI R. et al.** *Bioinformatics,* 2009, vol. 25 (15), 1966-7
- **ONDOV B.D. et al.** *Bioinformatics,* 2008, vol. 24 (23), 2776-7
- **NING Z. et al.** *Genome Res.,* 2001, vol. 11 (10), 1725-9
- **LUNTER G. ; GOODSON M.** *Genome Res.,* 2010